(19) Europäisches Patentamt
European Patent Office
Office européen des brevets

(11) **EP 3 909 437 A1**

(12) **EUROPEAN PATENT APPLICATION**
published in accordance with Art. 153(4) EPC

(43) Date of publication:
**17.11.2021 Bulletin 2021/46**

(51) Int Cl.:
*A23L 5/00* *(2016.01)*  *A23L 7/10* *(2016.01)*
*A23L 7/109* *(2016.01)*  *A23L 19/10* *(2016.01)*
*A23L 19/12* *(2016.01)*  *A23L 29/00* *(2016.01)*
*A21D 2/26* *(2006.01)*  *A21D 8/04* *(2006.01)*
*C12N 9/10* *(2006.01)*

(21) Application number: 20738732.5

(22) Date of filing: **10.01.2020**

(86) International application number:
**PCT/JP2020/000558**

(87) International publication number:
**WO 2020/145371 (16.07.2020 Gazette 2020/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.01.2019 JP 2019002862
29.03.2019 JP 2019069262**

(71) Applicant: **AJINOMOTO CO., INC.
Chuo-ku
Tokyo 104-8315 (JP)**

(72) Inventors:
• **AKAMOTO, Kazuto**
**Kawasaki-shi, Kanagawa 210-8681 (JP)**
• **SUGINO, Kazumi**
**Kawasaki-shi, Kanagawa 210-8681 (JP)**
• **SATO, Miho**
**Kawasaki-shi, Kanagawa 210-8681 (JP)**
• **YOKOYAMA, Noriko**
**Kawasaki-shi, Kanagawa 210-8681 (JP)**
• **SEKITA, Misa**
**Kawasaki-shi, Kanagawa 210-8681 (JP)**

(74) Representative: **Strehl Schübel-Hopf & Partner
Maximilianstrasse 54
80538 München (DE)**

(54) **METHOD FOR MANUFACTURING STARCH-CONTAINING FOOD**

(57) The present invention aims to provide a method for modifying a starch-containing food.

The present invention relates to a method for producing a starch-containing food, including adding (A) an amylomaltase derived from a bacterium of the genus Thermus to the starch-containing material, and further adding (B) a protein or lipid modification enzyme, or (C) a starch degradation product or (D) a starch degradation enzyme to the starch-containing material.

**Description**

[Technical Field]

The present invention relates to a method for producing a starch-containing food. The present invention also relates to a method for modifying a starch-containing food (e.g., a method for suppressing retrogradation of starch-containing foods, a method for improving texture of starch-containing foods, a method for improving production suitability of starch-containing foods, a method for improving flavor of starch-containing foods, etc.). Furthermore, the present invention relates to an enzyme composition that is preferably used for these methods.

[Background Art]

It is known that starch in starch-containing foods such as bakery food, rice food and the like retrogradates during storage and the quality (e.g., texture, etc.) of the foods deteriorates over time. Various methods have conventionally been proposed to improve the quality of starch-containing foods by suppressing such retrogradation of the starch.
For example, there have been proposed reducing the molecular weight of starch in foods with amylomaltase that has heat resistance and does not substantially catalyze hydrolysis and producing cyclic glucan in order to achieve food modification (preventing retrogradation of starch, etc.) (patent document 1); allowing 4-$\alpha$-glucanotransferase to act on starch granules under the condition where the starch granules do not dissolve, in order to obtain starch granules with improved retrogradation property, especially, starch granules with less progress of retrogradation over time (patent document 2); adding an appropriate amount of a bread dough improving composition containing at least one kind of lipase and at least one kind of glucose oxidase at the time of making bread dough, in order to produce satisfactory breads even when using a production line for large-scale production since the dough has sufficient mechanical resistance even without using chemical synthetic additives, to suppress retrogradation of bread, and to provide a soft, moist and voluminous bread (patent document 3); using a fat and oil composition containing glucose oxidase combined with protease at a specific ratio, in order to improve the freezing and thawing resistance of bread dough, and to produce frozen dough for making bread superior in texture, appearance and resistance to retrogradation, and having a high commercial value (patent document 4); and the like. It has also been reported that oligosaccharides have a retrogradation preventing effect on starchy foods (non-patent document 1). However, these methods may fail to afford a sufficient retrogradation suppressive effect, or these methods may suppress retrogradation but may impair good texture, flavor, production suitability, and the like of foods.
On the other hand, non-patent document 2 describes that the starch sugar transfer activity of amylomaltase derived from Corynebacterium glutamicum was measured by an iodine method using soluble potato starch and maltose, and amylomaltase is known to transfer the side chain of starch to maltose. However, no findings have been reported regarding the retrogradation suppressive effect of the reaction.

[Document List]

[Patent documents]

> patent document 1: JP-A-H11-46780
> patent document 2: WO 2012/111326
> patent document 3: JP-A-2002-272357
> patent document 4: JP-A-H11-332452

[Non-patent documents]

> non-patent document 1: Journal of Applied Glycoscinece (2011), vol.1, No.4, p. 281 - 285
> non-patent document 2: Applied and Environmental Microbiology (2012), vol.78, No.20, p.7223 - 7228

[Summary of Invention]

[Technical Problem]

The present invention has been made in view of the aforementioned situation, and aims to provide novel methods for modifying a starch-containing food (e.g., a novel method for suppressing retrogradation of a starch-containing food, a novel method for suppressing retrogradation and improving texture of a starch-containing food, a novel method for improving production suitability and texture of a starch-containing food, a novel method for improving flavor of a starch-containing food, etc.).

[Solution to Problem]

The present inventors have conducted intensive studies in an attempt to solve the aforementioned problems and found that a starch-containing food can be effectively modified (e.g., retrogradation of the starch-containing food can be effectively suppressed, retrogradation of the starch-containing food can be effectively suppressed and texture can be improved, production suitability of the starch-containing food can be effectively improved, texture can be improved, flavor of the starch-containing food can be improved, etc.) by adding amylomaltase derived from a bacterium of the genus Thermus which is combined with a protein or lipid modification enzyme, or a starch degradation product or a starch degradation enzyme to the starch-containing material. They have conducted further studies and completed the present invention.

Accordingly, the present invention provides the following.

[1] A method for producing a starch-containing food, comprising adding (A) an amylomaltase derived from a bacterium of the genus Thermus to the starch-containing material, and

further adding (B) a protein or lipid modification enzyme, or (C) a starch degradation product or (D) a starch degradation enzyme to the starch-containing material.

[2] The production method of [1], wherein an amount of the aforementioned (A) to be added to the starch-containing material is 0.0001 - 1000U per 1 g of the starch-containing material.

[3] The production method of [1], wherein an amount of the aforementioned (A) to be added to the starch-containing material is 0.01 - 20U (preferably, 0.05 - 20U) per 1 g of the starch-containing material.

[4] The production method of any one of [1] to [3], wherein the bacterium of the genus Thermus is at least one selected from the group consisting of Thermus thermophilus [alias, Thermus aquaticus, Thermus flavus], Thermus lacteus, Thermus rubens and Thermus ruber.

[5] The production method of any one of [1] to [4], wherein the protein or lipid modification enzyme is at least one selected from the group consisting of glucose oxidase, transglutaminase, protease, protein asparaginase, lipase and phospholipase.

[6] The production method of any one of [1] to [4], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[7] The production method of any one of [1] to [6], wherein the starch degradation product is at least one selected from the group consisting of glucose, maltose, maltotriose and dextrin, and

the starch degradation enzyme is at least one selected from the group consisting of $\alpha$-amylase, $\beta$-amylase, glucoamylase and $\alpha$-glucosidase.

[8] The production method of any one of [1] to [7], further comprising adding (E) a protein modification enzyme to the starch-containing material in addition to the aforementioned (A) , and (C) or (D) .

[9] The production method of [8], wherein the protein modification enzyme is at least one selected from the group consisting of glucose oxidase and transglutaminase.

[10] The production method of any one of [1] to [9], wherein the starch-containing food is selected from the group consisting of bakery food, rice food, potato food, dough sheet food and rice-cakes (preferably, the group consisting of bakery food, rice food, potato food and dough sheet food).

[11] An enzyme composition comprising (A) an amylomaltase derived from a bacterium of the genus Thermus, and (B) a protein or lipid modification enzyme, or (C) a starch degradation product or (D) a starch degradation enzyme.

[12] The enzyme composition of [11] for use for a starch-containing food.

[13] The enzyme composition of [12] for addition to the starch-containing material such that an amount of the aforementioned (A) to be added to the starch-containing material is 0.0001 - 1000U per 1 g of the starch-containing material.

[14] The enzyme composition of [12] for addition to the starch-containing material such that an amount of the aforementioned (A) to be added to the starch-containing material is 0.01 - 20U (preferably, 0.05 - 20U) per 1 g of the starch-containing material.

[15] The enzyme composition of any one of [11] to [14], wherein the bacterium of the genus Thermus is at least one selected from the group consisting of Thermus thermophilus [alias, Thermus aquaticus, Thermus flavus], Thermus lacteus, Thermus rubens and Thermus ruber.

[16] The enzyme composition of any one of [11] to [15], wherein the protein or lipid modification enzyme is at least one selected from the group consisting of glucose oxidase, transglutaminase, protease, protein asparaginase, lipase and phospholipase.

[17] The enzyme composition of any one of [11] to [15], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[18] The enzyme composition of any one of [11] to [17], wherein the starch degradation product is at least one selected from the group consisting of glucose, maltose, maltotriose and dextrin, and

the starch degradation enzyme is at least one selected from the group consisting of $\alpha$-amylase, $\beta$-amylase, glucoamylase and $\alpha$-glucosidase.

[19] The enzyme composition of any one of [11] to [18], further comprising (E) a protein modification enzyme in addition to the aforementioned (A), and (C) or (D).

[20] The enzyme composition of [19], wherein the protein modification enzyme is at least one selected from the group consisting of glucose oxidase and transglutaminase.

[21] The enzyme composition of any one of [12] to [20], wherein the starch-containing food is selected from the group consisting of bakery food, rice food, potato food, dough sheet food and rice-cakes (preferably, the group consisting of bakery food, rice food, potato food and dough sheet food).

[22] The enzyme composition of any one of [12] to [21] for modifying a starch-containing food.

[23] The enzyme composition of any one of [12] to [22] for suppressing retrogradation of the starch-containing food.

[24] The enzyme composition of any one of [12] to [23] for improving texture of the starch-containing food.

[25] The enzyme composition of any one of [12] to [24] for improving production suitability of the starch-containing food.

[26] The enzyme composition of any one of [12] to [25] for improving a flavor of the starch-containing food.

[27] A method for modifying a starch-containing food, comprising adding (A) an amylomaltase derived from a bacterium of the genus Thermus to the starch-containing material, and

further adding (B) a protein or lipid modification enzyme, or (C) a starch degradation product or (D) a starch degradation enzyme to the starch-containing material.

[28] The method of [27], wherein an amount of the aforementioned (A) to be added to the starch-containing material is 0.0001 - 1000U per 1 g of the starch-containing material.

[29] The method of [27], wherein an amount of the aforementioned (A) to be added to the starch-containing material is 0.01 - 20U (preferably, 0.05 - 20U) per 1 g of the starch-containing material.

[30] The method of any one of [27] to [29], wherein the bacterium of the genus Thermus is at least one selected from the group consisting of Thermus thermophilus [alias, Thermus aquaticus, Thermus flavus], Thermus lacteus, Thermus rubens and Thermus ruber.

[31] The method of any one of [27] to [30], wherein the protein or lipid modification enzyme is at least one selected from the group consisting of glucose oxidase, transglutaminase, protease, protein asparaginase, lipase and phospholipase.

[32] The method of any one of [27] to [30], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[33] The method of any one of [27] to [32], wherein the starch degradation product is at least one selected from the group consisting of glucose, maltose, maltotriose and dextrin, and

the starch degradation enzyme is at least one selected from the group consisting of $\alpha$-amylase, $\beta$-amylase, glucoamylase and $\alpha$-glucosidase.

[34] The method of any one of [27] to [33], further comprising adding (E) a protein modification enzyme to the starch-containing material in addition to the aforementioned (A), and (C) or (D) .

[35] The method of [34], wherein the protein modification enzyme is at least one selected from the group consisting of glucose oxidase and transglutaminase.

[36] The method of any one of [27] to [35], wherein the starch-containing food is selected from the group consisting of bakery food, rice food, potato food, dough sheet food and rice-cakes (preferably, the group consisting of bakery food, rice food, potato food and dough sheet food).

[37] The method of any one of [27] to [36], wherein the method for modifying a starch-containing food is at least one selected from the group consisting of a method for suppressing retrogradation of a starch-containing food, a method for improving texture of a starch-containing food, a method for improving production suitability of a starch-containing food, a method for improving a flavor of a starch-containing food and a method for modifying a property of a starch-containing food.

[38] A method for suppressing retrogradation of a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

[39] The method of [38], wherein an amount of the aforementioned (A) to be added to the starch-containing material is 0.0001 - 1000U per 1 g of the starch-containing material.

[40] The method of [38], wherein an amount of the aforementioned (A) to be added to the starch-containing material is 0.01 - 20U (preferably, 0.05 - 20U) per 1 g of the starch-containing material.

[41] The method of any one of [38] to [40], wherein the bacterium of the genus Thermus is at least one selected from the group consisting of Thermus thermophilus [alias, Thermus aquaticus, Thermus flavus], Thermus lacteus, Thermus rubens and Thermus ruber.

[42] The method of any one of [38] to [41], wherein the protein or lipid modification enzyme is at least one selected from the group consisting of glucose oxidase, transglutaminase, protease, protein asparaginase, lipase and phospholipase.

[43] The method of any one of [38] to [41], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[44] The method of any one of [38] to [43], wherein the starch degradation product is at least one selected from the group consisting of glucose, maltose, maltotriose and dextrin, and
the starch degradation enzyme is at least one selected from the group consisting of α-amylase, β-amylase, glucoamylase and α-glucosidase.

[45] The method of any one of [38] to [44], further comprising adding (E) a protein modification enzyme to the starch-containing material in addition to the aforementioned (A), and (C) or (D).

[46] The method of [45], wherein the protein modification enzyme is at least one selected from the group consisting of glucose oxidase and transglutaminase.

[47] The method of any one of [38] to [46], wherein the starch-containing food is selected from the group consisting of bakery food, rice food, potato food, dough sheet food and rice-cakes (preferably, the group consisting of bakery food, rice food, potato food and dough sheet food).

[48] The method of any one of [38] to [47], that is also a method for improving texture of a starch-containing food.

[49] A method for improving texture of a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

[50] The method of [49], wherein an amount of the aforementioned (A) to be added to the starch-containing material is 0.0001 - 1000U per 1 g of the starch-containing material.

[51] The method of [49], wherein an amount of the aforementioned (A) to be added to the starch-containing material is 0.01 - 20U (preferably, 0.05 - 20U) per 1 g of the starch-containing material.

[52] The method of any one of [49] to [51], wherein the bacterium of the genus Thermus is at least one selected from the group consisting of Thermus thermophilus [alias, Thermus aquaticus, Thermus flavus], Thermus lacteus, Thermus rubens and Thermus ruber.

[53] The method of any one of [49] to [52], wherein the protein or lipid modification enzyme is at least one selected from the group consisting of glucose oxidase, transglutaminase, protease, protein asparaginase, lipase and phospholipase.

[54] The method of any one of [49] to [52], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[55] The method of any one of [49] to [54], wherein the starch degradation product is at least one selected from the group consisting of glucose, maltose, maltotriose and dextrin, and
the starch degradation enzyme is at least one selected from the group consisting of α-amylase, β-amylase, glucoamylase and α-glucosidase.

[56] The method of any one of [49] to [55], further comprising adding (E) a protein modification enzyme to the starch-containing material in addition to the aforementioned (A), and (C) or (D).

[57] The method of [56], wherein the protein modification enzyme is at least one selected from the group consisting of glucose oxidase and transglutaminase.

[58] The method of any one of [49] to [57], wherein the starch-containing food is selected from the group consisting of bakery food, rice food, potato food, dough sheet food and rice-cakes (preferably, the group consisting of bakery food, rice food, potato food and dough sheet food).

[59] The method of any one of [49] to [58], that is also a method for suppressing retrogradation of a starch-containing food.

[60] The method of any one of [49] to [59], that is also a method for improving production suitability of a starch-containing food.

[61] A method for improving production suitability of a starch-containing food, comprising adding (A) an amylomaltase derived from a bacterium of the genus Thermus, and (B) a protein or lipid modification enzyme to the starch-containing material.

[62] The method of [61], wherein an amount of the aforementioned (A) to be added to the starch-containing material is 0.0001 - 1000U per 1 g of the starch-containing material.

[63] The method of [61], wherein an amount of the aforementioned (A) to be added to the starch-containing material is 0.01 - 20U (preferably, 0.05 - 20U) per 1 g of the starch-containing material.

[64] The method of any one of [61] to [63], wherein the bacterium of the genus Thermus is at least one selected from the group consisting of Thermus thermophilus [alias, Thermus aquaticus, Thermus flavus], Thermus lacteus, Thermus rubens and Thermus ruber.

[65] The method of any one of [61] to [64], wherein the protein or lipid modification enzyme is at least one selected from the group consisting of glucose oxidase, transglutaminase, protease, protein asparaginase, lipase and phospholipase.

[66] The method of any one of [61] to [64], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[67] The method of any one of [61] to [66], wherein the starch-containing food is selected from the group consisting of bakery food, rice food, potato food, dough sheet food and rice-cakes (preferably, the group consisting of bakery food, rice food, potato food and dough sheet food).

[68] The method of any one of [61] to [67], that is also a method for improving texture of a starch-containing food.

[69] A method for improving a flavor of a starch-containing food, comprising adding (A) an amylomaltase derived from a bacterium of the genus Thermus, and (B) a protein or lipid modification enzyme to the starch-containing material.

[70] The method of [69], wherein an amount of the aforementioned (A) to be added to the starch-containing material is 0.0001 - 1000U per 1 g of the starch-containing material.

[71] The method of [69], wherein an amount of the aforementioned (A) to be added to the starch-containing material is 0.01 - 20U (preferably, 0.05 - 20U) per 1 g of the starch-containing material.

[72] The method of any one of [61] to [71], wherein the bacterium of the genus Thermus is at least one selected from the group consisting of Thermus thermophilus [alias, Thermus aquaticus, Thermus flavus], Thermus lacteus, Thermus rubens and Thermus ruber.

[73] The method of any one of [69] to [72], wherein the protein or lipid modification enzyme is at least one selected from the group consisting of glucose oxidase, transglutaminase, protease, protein asparaginase, lipase and phospholipase.

[74] The method of any one of [69] to [72], wherein the protein or lipid modification enzyme is at least one selected from the group consisting of glucose oxidase, transglutaminase, protease, protein asparaginase and lipase (preferably, at least one selected from the group consisting of glucose oxidase, transglutaminase, protease and lipase).

[75] The method of any one of [69] to [74], wherein the starch-containing food is selected from the group consisting of bakery food, rice food, potato food, dough sheet food and rice-cakes (preferably, the group consisting of bakery food, rice food, potato food and dough sheet food).

[Advantageous Effects of Invention]

According to the present invention, methods for producing a modified starch-containing food (e.g., a method for producing a starch-containing food with suppressed retrogradation, a method for producing a starch-containing food with suppressed retrogradation and improved texture, a method for producing a starch-containing food with improved production suitability and improved texture, a method for producing a starch-containing food with improved flavor, etc.) can be provided.

In one embodiment according to the present invention, retrogradation of a starch-containing food can be suppressed, and therefore, according to the present invention, a method for producing a starch-containing food with suppressed retrogradation and a method for suppressing retrogradation of a starch-containing food can be provided.

In another embodiment according to the present invention, retrogradation of a starch-containing food can be suppressed, and texture can be improved. According to the present invention, therefore, a method for producing a starch-containing food with suppressed retrogradation and improved texture, a method for suppressing retrogradation of a starch-containing food, and a method for improving texture of a starch-containing food can be provided.

In another embodiment according to the present invention, production suitability of a starch-containing food can be improved and the texture can be improved. According to the present invention, therefore, a method for producing a starch-containing food with improved production suitability and improved texture, and a method for improving production suitability of a starch-containing food can be provided.

In another embodiment according to the present invention, a method for producing a starch-containing food with improved flavor, and a method for improving a flavor of a starch-containing food can be provided.

According to the present invention, an enzyme composition that can be preferably used for modifying a starch-containing food can be provided.

In one embodiment according to the present invention, an enzyme composition that can be preferably used for suppressing retrogradation of a starch-containing food and/or improving texture of a starch-containing food can be provided.

In another embodiment according to the present invention, an enzyme composition that can be preferably used for improving production suitability of a starch-containing food and/or improving texture of a starch-containing food can be provided.

In another embodiment according to the present invention, an enzyme composition that can be preferably used for improving a flavor of a starch-containing food can be provided.

[Brief Description of Drawings]

Fig. 1 shows a purification flow of the amylomaltase derived from Thermus thermophilus used in the Examples.

Fig. 2 is a graph showing the stress (N) of the white breads of samples 2-1, 2-3, 2-7 and 2-9 measured in Experimental Example 3 at a compression ratio of 10%.

Fig. 3 is a graph showing the stress (N) of the white breads of samples 4-1 to 4-4 measured in Experimental Example 4 at a compression ratio of 10%.

Fig. 4 is a graph showing the stress (N) of the white breads of samples 5-1 to 5-4 measured in Experimental Example 5 at a compression ratio of 10%.

Fig. 5 is a graph showing the hardness of the mashed potato of Experimental plots 7-1 to 7-6 and Comparison plots 7-1 to 7-5.

Fig. 6 is a graph showing the hardness of the white breads of Experimental plot 8-1 and Experimental plot 8-2, and Comparison plots 8-1 to 8-4.

Fig. 7 is a graph showing the hardness of the white breads of Experimental plot 9-1 and Experimental plot 9-2, and Comparison plot 9-1 and Comparison plot 9-2.

Fig. 8 is a graph showing the hardness of the mashed potato of Experimental plot 11-1 and Comparison plots 11-1 to 11-3.

Fig. 9 is a graph showing the stress (N) of the white breads of Experimental plot 12-1 and Comparison plots 12-1 to 12-3 measured in Experimental Example 14 at a compression ratio of 10%.

Fig. 10 is a graph showing the hardness of the mashed potato of Experimental plot 13-1 and Comparison plots 13-1 to 13-3.

[Description of Embodiments]

1. Production method of starch-containing food

One of the characteristics of the method for producing a starch-containing food of the present invention (sometimes to be referred to as "the production method of the present invention" in the present specification) is that it includes adding (i) (A) an amylomaltase derived from a bacterium of the genus Thermus (sometimes to be simply referred to as "(A)" in the present specification) to a starch-containing material, and further adding (ii) (B) a protein or lipid modification enzyme (sometimes to be simply referred to as "(B)" in the present specification), or (C) a starch degradation product (sometimes to be simply referred to as "(C)" in the present specification) or (D) a starch degradation enzyme (sometimes to be simply referred to as "(D)" in the present specification) to the starch-containing material.

In the present invention, the "starch-containing food" refers to a food produced using a starch-containing material as one of the materials. Examples thereof include, but are not limited to, bakery food (e.g., bread, cake, cookie, etc.), rice food (e.g., cooked rice, fried rice, rice ball, etc.), potato food (e.g., mashed potato, french fries, potato salad, potato flakes, etc.), dough sheet food (e.g., Japanese wheat noodles, Chinese noodles, gyoza, etc.), rice-cakes (e.g., rice-cake, rice-flour dumpling, arrowroot-starch cake, etc.) and the like.

In the present invention, the "bakery food" refers to a food obtained by heating (e.g., baking, etc.) a dough obtained by adding water or the like to flour (e.g., wheat flour, etc.) and kneading same. Specific examples include, but are not limited to, bread, cake, cookie and the like. The "rice food" refers to a food made from rice as one of the materials. Specific examples include, but are not limited to, cooked rice, fried rice, rice ball and the like. The "potato food" refers to a food made from potatoes (e.g., potato, etc.) as one of the materials. Specific examples include, but are not limited to, mashed potato, french fries, potato salad, potato flakes and the like. The "dough sheet food" refers to a food made from a dough sheet as one of the materials. The "dough sheet" here refers to a food material obtained by rolling (e.g., band-like, sheet-like, etc.) thinly a dough obtained by adding water or the like to flour (e.g., wheat flour, etc.) and kneading same. Specific examples of the dough sheet food include, but are not limited to, Japanese wheat noodles, Chinese noodles, gyoza and the like. The "rice-cakes" refers to a food material obtained by molding a dough obtained by adding water or the like to starch (e.g., rice flour, top-grade non-glutinous rice flour, arrowroot flour, etc.) and kneading same. Specific examples of the rice-cakes include, but are not limited to, rice-cake, rice-flour dumpling, arrowroot-starch cake and the like.

[(A) Amylomaltase derived from bacterium of the genus Thermus]

In the present invention, the "amylomaltase" is an enzyme that catalyzes a chemical reaction that transfers a portion of $\alpha$-glucan chain from a non-reducing terminal of $\alpha$-glucan (e.g., amylose, amylopectin, starch, etc.) to a non-reducing terminal of another $\alpha$-glucan (or glucose). The donor molecule and the receptor molecule of $\alpha$-glucan chain may be the same. In this case, an intramolecular transfer is caused, and the resultant product has a cyclic structure. Amylomaltase is classified into enzyme commission number EC2.4.1.25 determined by the International Union of Biochemistry and Molecular Biology (IUBMB), and is generally referred to as 4-$\alpha$-glucanotransferase or the like.

The amylomaltase to be used in the present invention is preferably derived from a bacterium of the genus Thermus. Amylomaltase derived from a bacterium of the genus Thermus can retain high activity at about 60 - 80°C and an enzymatic reaction can proceed during the production and cooking of starch-containing foods. In addition, amylomaltase derived from a bacterium of the genus Thermus can be easily deactivated by heating to about 90 - 100°C.

Examples of the bacterium of the genus Thermus that can produce amylomaltase include Thermus thermophilus [alias, Thermus aquaticus, Thermus flavus], Thermus lacteus, Thermus rubens, Thermus ruber and the like. The species of the amylomaltase used in the present invention is not particularly limited as long as it is derived from a bacterium of the genus Thermus. It is preferably Thermus thermophilus. These bacteria are easily available from a depositary institution and the like. In the present invention, the bacterium of the genus Thermus may be a wild-type, or a variant.

The "amylomaltase derived from bacterium of the genus Thermus" in the present invention includes, in addition to an amylomaltase produced by bacterium of the genus Thermus, an amylomaltase obtained by a genetic engineering method using a gene encoding the amylomaltase produced by bacterium of the genus Thermus. For example, an amylomaltase expressed by a host (e.g., Escherichia coli, etc.) transformed or transduced with a gene encoding amylomaltase produced by a bacterium of the genus Thermus, and the like are also encompassed in the "amylomaltase derived from bacterium of the genus Thermus" in the present invention. the amino acid sequence of amylomaltase derived from a bacterium of the genus Thermus, and the base sequence of the gene encoding the amylomaltase can be obtained from a public database such as the National Center for Biotechnology Information (NCBI) and the like.

The production method of (A) is not particularly limited, and (A) can be produced by a method known per se (e.g., the method described in JP-A-H11-46780, the method described in Thijs Kaper et al., Biochemistry, 2007, vol.46, pp.5261-5269, etc.) or a method analogous thereto. For example, (A) can be produced by culturing bacteria of the genus Thermus capable of producing amylomaltase and recovering amylomaltase from the obtained culture. The culture conditions of the bacteria of the genus Thermus are not particularly limited as long as the bacteria of the genus Thermus can grow and amylomaltase is produced. It can also be produced by the method used in the Examples described later or a method similar thereto.

(A) may be purified to a desired degree, or a culture of a bacterium of the genus Thermus that produces amylomaltase, a bacterial cell separated from the culture, a treated product of the bacterial cell, or the like may also be used. When (A) is a purified product, the purification method is not particularly limited, and a method known per se or a method analogous thereto may be used. For example, purification can be performed by the method used in the Examples described later or a method analogous thereto.

A variant of an amylomaltase derived from a bacterium of the genus Thermus may be used for (A). In addition, a commercially available product may also be used for (A).

In the present invention, the activity unit of amylomaltase is measured and defined as follows.

A reaction mixture (1 mL) of 0.05% solubilized starch, 0.05% maltose, 30 mM sodium acetate buffer (pH 5.5) and an enzyme liquid (0.01 mL) is allowed to react at 70°C for 5 min, and heated at 96°C for 5 min to discontinue the reaction. Thereafter, 0.1 mL of the reaction mixture is mixed with 1 mL of an iodine solution (0.02% $I_2$, 0.2% KI), and the absorbance at 600 nm is measured. A value obtained by subtracting the measured value when the enzyme was used from the measured value when the enzyme was not used (control) is defined as the activity value. The amount of enzyme necessary for reducing the absorbance at 600 nm by 1 per minute is defined as 1U (unit) .

[(B) Protein or lipid modification enzyme]

In the present invention, the "protein or lipid modification enzyme" is an enzyme having an activity of acting on a protein or lipid and modifying same (e.g., crosslinking, degradation, etc.). For example, protein modification enzymes such as protein crosslinking enzymes (e.g., glucose oxidase, transglutaminase, etc.), protein degradation enzymes (protease), other protein modify enzymes (e.g., protein asparaginase, etc.) and the like; lipid modification enzymes such as lipid degrading enzymes (e.g., lipase, phospholipase, etc.) and the like, and the like can be mentioned. Preferred are glucose oxidase, transglutaminase, protease, protein asparaginase, lipase, and phospholipase, more preferred are glucose oxidase, transglutaminase, protease, and lipase, and particularly preferred are glucose oxidase, transglutaminase, and protease.

Glucose oxidase (enzyme commission number EC1.1.3.4) is an oxidase that catalyzes the reaction that produces gluconic acid and hydrogen peroxide using glucose, oxygen, and water as substrates. The hydrogen peroxide produced by this reaction promotes the formation of SS bond (disulfide bond) by oxidizing SH groups in a protein, and forms a crosslinked structure in the protein. Glucose oxidases of various origins are known such as those derived from microorganism, plant, and the like. The enzyme used in the present invention may be any enzyme as long as it has the aforementioned activity and its origin is not limited. It may also be a recombinant enzyme. The glucose oxidase to be used in the present invention may be a commercially available product, and one example is glucose oxidase derived from the microorganism commercially available from Nippon Chemical Industrial Co., Ltd. under the trade name "Sumizyme PGO". A glucose oxidase preparation containing catalase is commercially available, and the glucose oxidase used in the present invention may be a mixture with other enzyme as long as it has glucose oxidase activity.

In the present invention, the activity unit of the glucose oxidase can be measured and defined as follows.

Using glucose as a substrate, glucose oxidase is reacted in the presence of oxygen to produce hydrogen peroxide. The produced hydrogen peroxide is reacted with peroxidase in the presence of aminoantipyrine and phenol. The color tone exhibited by the produced quinoneimine dye is measured and quantified at a wavelength of 500 nm. An enzyme amount necessary for oxidizing 1 pmol of glucose in 1 min is defined as 1U (unit).

Transglutaminase (enzyme commission number EC2.3.2.13) is an enzyme having an activity of catalyzing an acyl transfer reaction using a glutamine residue in a protein or peptide as a donor and a lysine residue as a receptor, and transglutaminases having various origins are known such as those derived from mammals, those derived from fish, those derived from microorganisms, and the like. The origin of the transglutaminase used in the present invention is not particularly limited as long as it has the aforementioned activity, and a transglutaminase of any origin can be used, and a recombinant enzyme may also be used. The transglutaminase used in the present invention may be a commercially available product. Specific examples thereof include transglutaminase derived from a microorganism and commercially available under the trade name of "Activa" (registered trade mark) TG from Ajinomoto Co., Inc., and the like.

In the present invention, the activity unit of transglutaminase can be measured and defined as follows.

Benzyloxycarbonyl-L-glutamylglycine and hydroxylamine as substrates are allowed to act on transglutaminase, and an iron complex of hydroxamic acid is formed in the presence of trichloroacetic acid. The absorbance at 525 nm is measured, the amount of the hydroxamic acid is determined from the calibration curve, and the enzyme activity is calculated. An enzyme amount necessary for producing 1 pmol of hydroxamic acid at 37°C, pH 6.0 in 1 min is defined as 1U (unit).

The protease used in the present invention (enzyme commission number EC3.4 group) is an enzyme that catalyzes the hydrolysis of peptide bond in a protein, and a protease having any substrate specificity and any reaction property can also be used in the present invention as long as it has the activity and is capable of degrading an animal-derived protein. The origin thereof is not particularly limited, and a protease of any origin can be used such as proteases derived from plants (e.g., bromelain, papain, actinidin, ficin, etc.), proteases derived from mammals (e.g., trypsin, cathepsin, etc.), proteases derived from fish, proteases derived from microorganisms (e.g., subtilisin, thermolysin, etc.) and the like, and a recombinant enzyme may also be used. Specific examples of the protease used in the present invention include "Bromelain F" (manufactured by Amano Enzyme Inc.), "purified papain for food" (manufactured by Nagase & Co., Ltd.), "protease A" (manufactured by Amano Enzyme Inc.), "Protin FN" (manufactured by Daiwa Fine Chemicals Co., Ltd.), "Protin SD-NY10" (manufactured by Amano Enzyme Inc.) and the like.

In the present invention, as the activity unit of protease, an enzyme amount necessary for increasing a Folin reagent color developed substance corresponding to 1 $\mu$g of tyrosine at $37\pm0.5$°C in 1 min by using casein as a substrate is defined as 1U (unit).

Protein asparaginase is an enzyme having an activity of catalyzing a deamidation reaction of an asparagine residue in a protein. A protein asparaginase derived from microorganism, and the like are known. The enzyme used in the present invention may be any enzyme as long as it has the aforementioned activity and its origin is not limited. It may also be a recombinant enzyme. The protein asparaginase used in the present invention is preferably derived from a microorganism. Examples of the protein asparaginase derived from a microorganism include a protein asparaginase derived from a bacterium of genus Leifsonia (e.g., Leifsonia xyli, Leifsonia aquatica, etc.), a protein asparaginase derived from a bacterium of genus Microbacterium (e.g., Microbacterium testaceum, etc.), a protein asparaginase derived from a bacterium of genus Luteimicrobium (e.g., Luteimicrobium album, etc.), a protein asparaginase derived from a bacterium of genus Agromyces (e.g., Agromyces sp., etc.) and the like. The protein asparaginase used in the present invention may be a commercially available product.

In the present invention, the activity unit of the protein asparaginase can be measured and defined as follows.

An enzyme solution (25 $\mu$L) having an appropriate concentration is added to a 0.2 mol/L phosphate buffer (pH 6.5) (125 $\mu$L) containing 30 mmol/L Cbz-Asn-Gly, and incubated at 37°C for 60 min. A 12% trichloroacetic acid solution (150 $\mu$L) is added to discontinue the reaction, and the ammonia concentration of the supernatant is measured. An enzyme amount necessary for producing 1 $\mu$mol of ammonia in 1 min is defined as 1U (unit).

Lipase is an enzyme having an activity of hydrolyzing glycerol fatty acid ester into fatty acid and glycerol and encompasses, for example, triacylglycerol lipase, triacylglyceride lipase and the like. Lipases of various origins are known such as those derived from microorganism, plant, animal, and the like. The lipase used in the present invention may be of any origin as long as it has the aforementioned activity and its origin is not limited. In addition, a recombinant enzyme may also be used. The lipase used in the present invention is preferably derived from a microorganism. Examples of the lipase derived from a microorganism include a lipase derived from a bacterium of genus Aspergillus (e.g., Aspergillus niger, etc.), a lipase derived from a bacterium of genus Rhizopus (e.g., Rhizopus oryzae, etc.), a lipase derived from a bacterium of genus Candida (e.g., Candida rugosa, etc.), a lipase derived from a bacterium of genus Penicillium (e.g., Penicillium camemberti, Penicillium roqueforti, etc.) and the like. The lipase used in the present invention may be a commercially available product, and specific examples thereof include Lipase GS "Amano" 250G, Lipase AY "Amano" 30SD, Lipase R "Amano", Lipase A "Amano" 6, Lipase MER "Amano" (all manufactured by Amano Enzyme Inc.), "Lilipase A-10D" (manufactured by Yakult Pharmaceutical Industry Co., Ltd.) and the like.

In the present invention, the activity unit of lipase can be measured and defined as follows.

Olive oil (25 mL) and 2% PVA reagent solution (75 mL) are emulsified to give a substrate. The substrate (5 mL) is blended with McIlvaine buffer (pH 7.0) (4 mL) and an enzyme liquid (1 mL), reacted at 37°C for 30 min, and the reaction is discontinued. The resulting fatty acid is measured by a titration method. An enzyme amount necessary for liberating an acid corresponding to 1 $\mu$mol of liberated fatty acid is defined as 1U (unit).

Phospholipase is an enzyme having an activity of hydrolyzing phospholipids, and it is classified into $A_1$, $A_2$, C and D according to the binding position to be hydrolyzed. The phospholipase used in the present invention is not particularly limited as long as it has the aforementioned activity, and any phospholipase can also be used. Preferred are phospholipase D, phospholipase $A_2$. As the phospholipase, a recombinant enzyme may be used. The phospholipase used in the present invention may be a commercially available product, and specific examples thereof include Denabake RICH, PLA2 NAGASE 10P/R, PLA2 NAGASE L/R (all manufactured by Nagase ChemteX Corporation) and the like.

In the present invention, the activity unit of the phospholipase D can be measured and defined as follows.

A substrate solution (0.9 mL) containing phosphatidyl choline is blended with an enzyme solution (0.1 mL) and the mixture is reacted at 37°C for 30 min. After discontinuation of the reaction, the reaction mixture (50 $\mu$L) is added to a color development solution (1 mL) containing cholinesterase and allowed to react for 5 min. After discontinuation of the reaction, the amount of dye produced by choline is measured. An enzyme amount necessary for liberating 1 pmol of choline at 37°C in 1 min by using phosphatidyl choline as a substrate is defined as 1U (unit).

Also, in the present invention, the activity unit of phospholipase $A_1$, phospholipase $A_2$ can be measured and defined as follows.

The activity that liberates an acid corresponding to 1 $\mu$mol using egg-yolk (containing about 0.4% phospholipid) as a substrate at pH 8, 40°C for 1 min is defined as 1U.

[(C) Starch degradation product]

In the present invention, the "starch degradation product" refers to a food material that can be obtained by reducing the molecular weight (hydrolysis) of starch (including processed starch) by using an enzyme and/or an acid. The DE of the starch degradation product that can be used in the present invention is preferably 3 - 100, more preferably 5 - 100, particularly preferably 30 - 100, most preferably 40 - 100. Specific examples starch degradation product that can be used in the present invention include glucose (DE:100); maltooligosaccharides having a degree of polymerization of 2 - 7 (preferably 2 - 5) such as maltose (DE:50), maltotriose (DE:33), maltotetraose (DE:25) and the like; corn syrup solids (DE: generally over 20 and not more than 40); dextrin (DE: not more than 20, preferably not more than 10) and the like. Preferred are glucose, maltooligosaccharides having a degree of polymerization of 2 - 7, dextrin with DE of not more than 20, more preferred are glucose, maltose, maltotriose, dextrin with DE of not more than 10, particularly preferred are glucose, maltose, and most preferred is maltose.

As used herein, "DE" is an abbreviation for Dextrose Equivalent, and is shown by the following formula. DE is used as an index showing the degree of hydrolysis of starch degradation products, and the closer the DE is to 100, the more the hydrolysis proceeds, indicating that the starch degradation product is closer to glucose.

```
DE=[direct reducing sugar (converted to glucose)/solid
content]×100
```

The production method of (C) that may be used in the present invention is not particularly limited, and (C) may be produced by a method known per se or a method analogous thereto. While (C) may be produced by, for example, hydrolyzing starch with an enzyme and/or an acid and the like, the production method of (C) is not limited to this method, and may be produced by other method. A commercially available product may also be used for (C).

[(D) Starch degradation enzyme]

In the present invention, the "starch degradation enzyme" is an enzyme having an activity of acting on and degrading starch. For example, α-amylase, β-amylase, glucoamylase, α-glucosidase and the like can be mentioned.

α-Amylase is an endo-type enzyme having an activity of hydrolyzing α-1,4-glucoside bond of starch at unspecified locations and, for example, those derived from microorganisms (e.g., fungus of genus Bacillus, fungus of genus Aspergillus, etc.) and the like are known. The origin of the α-amylase used in the present invention is not particularly limited as long as it has the aforementioned activity, α-amylase of any origin can be used, and a recombinant enzyme may also be used. The α-amylase used in the present invention may be a commercially available product and specific examples include Novamyl 10000 BG, Novamyl 3D BG (both manufactured by Novozymes Japan Ltd.), Biozyme A, Biozyme LC (both manufactured by Amano Enzyme Inc.) and the like.

In the present invention, the activity unit of α-amylase can be measured and defined as follows.

Blocked p-nitrophenyl maltoheptaoside as a substrate is reacted with α-amylase. Thereafter, the produced p-nitrophenyl maltosaccharide is degraded with heat-resistant α-glucosidase and the reaction is discontinued with trisodium phosphate. The absorbance of the produced p-nitrophenol at 410 nm is measured. An enzyme amount necessary for liberating 1 pmol of p-nitrophenol in 1 min is defined as 1U (unit).

β-Amylase is an exo-type enzyme having an activity of hydrolyzing α-1,4-glucoside bond of starch alternately (every other maltose unit) from the non-reducing terminal, and β-amylases of various origins are known such as those derived from microorganisms, those derived from plants, and the like. The origin of the β-amylase used in the present invention is not particularly limited as long as it has the aforementioned activity, β-amylase of any origin can be used, and a recombinant enzyme may also be used. The β-amylase used in the present invention may also be a commercially available product and specific examples thereof include Himaltosin GL, Himaltosin GLH (both manufactured by HBI Enzymes Inc.), β-amylase F "Amano" (manufactured by Amano Enzyme Inc.) and the like.

In the present invention, the activity unit of β-amylase can be measured and defined as follows.

p-Nitrophenyl-β-D-maltotrioside (PNP-β) as a substrate is reacted with β-amylase. Thereafter, the amount of the produced p-nitrophenol (PNP) is measured from the absorbance at 400 nm. An enzyme amount necessary for dissociating 1 pmol of PNP in 1 min is defined as 1U (unit).

Glucoamylase is an exo-type enzyme having an activity of hydrolyzing α-1,4-glucoside bond, α-1,6-glucoside bond of starch from non-reducing terminal to produce β-D-glucose and those derived from microorganisms (e.g., fungus of genus Aspergillus, fungus of genus Rhizopus, etc.) and the like are known. The origin of the glucoamylase used in the present invention is not particularly limited as long as it has the aforementioned activity, glucoamylase of any origin can be used, and a recombinant enzyme may also be used. The glucoamylase used in the present invention may be a commercially available product. Specific examples include AMG1100BG, AMG 300L (both manufactured by Novozymes Japan Ltd.), Sumizyme, Sumizyme S, Sumizyme SG (all manufactured by SHINNIHON CHEMICALS Corporation), glucoamylase for sake brewing "Amano" SD (manufactured by Amano Enzyme Inc.) and the like.

In the present invention, the activity unit of glucoamylase can be measured and defined as follows.

To a 2% soluble starch solution (1 mL) is added a 0.2M acetate buffer (pH 5.0) (0.2 mL) and the mixture is preheated at 40°C for 5 min. An enzyme liquid (0.1 mL) is added thereto and reacted at 40°C for 20 min. A 1N sodium hydroxide solution (0.1 mL) is added to discontinue the reaction. The mixture is left standing for 30 min, and neutralized with 1N hydrochloric acid (0.1 mL). The amount of glucose developed in the reaction mixture is quantified, and an enzyme amount necessary for producing 1 mg of glucose at 40°C in 60 min from soluble starch is defined as 1U (unit).

α-Glucosidase is an enzyme having an activity of hydrolyzing α-1,4-glucoside bond of saccharides and starch from the non-reducing terminal to produce α-glucose. The origin of the α-glucosideused in the present invention is not particularly limited as long as it has the aforementioned activity, α-glucosideof any origin can be used, and a recombinant enzyme may also be used. The α-glucosideused in the present invention may also be a commercially available product and specific examples thereof include α-Glucosidase "Amano", Transglucosidase L "Amano" (both manufactured by Amano Enzyme Inc.) and the like.

In the present invention, the activity unit of α-glucosidase can be measured and defined as follows.

To 1 mM α-methyl-D-glucoside (1 mL) is added 0.02M acetate buffer (pH 5.0) (1 mL), an enzyme solution (0.5 mL) is added, and the mixture is reacted at 40°C for 60 min. An enzyme amount necessary for producing 1 μg of glucose in 2.5 mL of the reaction mixture is defined as 1U (unit).

The production method of the present invention may further include adding (E) a protein modification enzyme to a starch-containing material, in addition to the addition of (A), and (C) or (D) to the starch-containing material.

In the present specification, the "protein modification enzyme" is sometimes to be referred to as "(E)" for convenience.

The "protein modification enzyme" that can be used as (E) in the present invention is an enzyme having an activity of acting on a protein and modifying same (e.g., crosslinking, degradation, etc.). For example, protein modification enzymes such as protein crosslinking enzymes (e.g., glucose oxidase, transglutaminase, etc.) can be mentioned. The "protein modification enzyme" (described above) that can be used as (B) in the present invention may also be used.

In the production method of the present invention, the amount of (A) to be added to the starch-containing material is preferably not less than 0.0001U, more preferably not less than 0.1U, particularly preferably not less than 0.5U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In addition, the amount of (A) to be added to the starch-containing material is preferably not more than 1000U, more preferably not more than 100U, particularly preferably not more than 20U, per 1 g of the starch-containing material, because a starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, in the production method of the present invention, the amount of (A) to be added to the starch-containing material is preferably not less than 0.01U, more preferably not less than 0.03U, particularly preferably not less than 0.05U, per 1 g of the starch-containing material, because retrogradation of the starch-containing food can be effectively suppressed. Also, in one embodiment, the amount of (A) to be added to the starch-containing material is preferably not more than 100U, more preferably not more than 20U, particularly preferably not more than 10U, per 1 g of the starch-containing material, because retrogradation of the starch-containing food can be effectively suppressed.

When the production method of the present invention includes adding (B) to a starch-containing material, the amount of (B) to be added to the starch-containing material is preferably not less than 0.00001U, more preferably not less than 0.0001U, particularly preferably not less than 0.0005U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In this case, the amount of (B) to be added to the starch-containing material is preferably not more than 1000U, more preferably not more than 100U, particularly preferably not more than 10U, per 1 g of the starch-containing material, because a starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, when the production method of the present invention includes adding (B) to a starch-containing material and, for example, glucose oxidase or the like is used as (B), the amount of (B) to be added to the starch-containing material is preferably not less than 0.0001U, more preferably not less than 0.001U, particularly preferably not less than 0.01U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In this case, the amount of (B) to be added to the starch-containing material is preferably not more than 1000U, more preferably not more than 1U, particularly preferably not more than 0.05U, per 1 g of the starch-containing material, because a starch-containing food can be effectively modified (e.g., retrogradation of the starch-containing food can be effectively suppressed, texture of the starch-containing food can be effectively improved, production suitability of the starch-con-

12

taining food can be effectively improved, a flavor of the starch-containing food can be improved, a property of the starch-containing food can be improved, etc.).

In another embodiment, when the production method of the present invention includes adding (B) to a starch-containing material and, for example, transglutaminase or the like is used as (B), the amount of (B) to be added to the starch-containing material is preferably not less than 0.00001U, more preferably not less than 0.0001U, particularly preferably not less than 0.0005U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In this case, the amount of (B) to be added to the starch-containing material is preferably not more than 1000U, more preferably not more than 1U, particularly preferably not more than 0.005U, per 1 g of the starch-containing material, because a starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In another embodiment, when the production method of the present invention includes adding (B) to a starch-containing material and, for example, lipase or the like is used as (B), the amount of (B) to be added to the starch-containing material is preferably not less than 0.001U, more preferably not less than 0.01U, particularly preferably not less than 0.1U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In this case, the amount of (B) to be added to the starch-containing material is preferably not more than 1000U, more preferably not more than 100U, particularly preferably not more than 50U, per 1 g of the starch-containing material, because a starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In another embodiment, when the production method of the present invention includes adding (B) to a starch-containing material and, for example, protease or the like is used as (B), the amount of (B) to be added to the starch-containing material is preferably not less than 0.00001U, more preferably not less than 0.0001U, particularly preferably not less than 0.001U, per 1 g of the starch-containing material, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.). In this case, the amount of (B) to be added to the starch-containing material is preferably not more than 1000U, more preferably not more than 100U, particularly preferably not more than 10U, per 1 g of the starch-containing material, because a starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, when the production method of the present invention includes adding (B) to a starch-containing material and, for example, glucose oxidase or the like is used as (B), the activity ratio of the amount of (A) and the amount of (B) to be added to the starch-containing material is preferably (A):(B)=1:0.0001 - 0.04, more preferably (A):(B)=1:0.0005 - 0.035, particularly preferably (A):(B)=1:0.001 - 0.03, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, when the production method of the present invention includes adding (B) to a starch-containing material and, for example, transglutaminase or the like is used as (B), the activity ratio of the amount of (A) and the amount of (B) to be added to the starch-containing material is preferably (A):(B)=1:0.000001 - 1000, more preferably (A):(B)=1:0.00001 - 0.01, particularly preferably (A):(B)=1:0.00015 - 0.003, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, when the production method of the present invention includes adding (B) to a starch-containing material and, for example, lipase or the like is used as (B), the activity ratio of the amount of (A) and the amount of (B) to be added to the starch-containing material is preferably (A):(B)=1:0.001 - 1000, more preferably (A):(B)=1:0.01 - 100, particularly preferably (A):(B)=1:0.1 - 50, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

In one embodiment, when the production method of the present invention includes adding (B) to a starch-containing material and, for example, protease or the like is used as (B), the activity ratio of the amount of (A) and the amount of (B) to be added to the starch-containing material is preferably (A):(B)=1:0.00001 - 1000, more preferably (A):(B)=1:0.0001 - 100, particularly preferably (A):(B)=1:0.01 - 10, because the starch-containing food can be effectively modified (e.g., retrogradation of the starch-containing food can be effectively suppressed, texture of the starch-containing food can be effectively improved, production suitability of the starch-containing food can be effectively improved, a flavor of the starch-containing food can be improved, a property of the starch-containing food can be improved, etc.).

When the production method of the present invention includes adding (C) to a starch-containing material, the amount of (C) to be added to the starch-containing material is preferably not less than 0.05 wt%, more preferably not less than 0.1 wt%, particularly preferably not less than 0.3 wt%, of the starch-containing material, because the action of (A) can be effectively enhanced. In this case, the amount of (C) to be added to the starch-containing material is preferably not more than 70 wt%, more preferably not more than 60 wt%, particularly preferably not more than 55 wt%, of the starch-containing material, because the action of (A) can be effectively enhanced.

In one embodiment, when the production method of the present invention includes adding (D) to a starch-containing material and, for example, $\alpha$-amylase or the like is used as (D), the amount of (D) to be added to the starch-containing material is preferably not less than 0.001U, more preferably not less than 0.01U, particularly preferably not less than 0.1U, per 1 g of the starch-containing material, from the aspect of the level of the retrogradation suppressive effect. In this case, the amount of (D) to be added to the starch-containing material is preferably not more than 100U, more preferably not more than 10U, particularly preferably not more than 1U, per 1 g of the starch-containing material, from the aspect of preference for the properties.

In another embodiment, when the production method of the present invention includes adding (D) to a starch-containing material and, for example, $\beta$-amylase or the like is used as (D), the amount of (D) to be added to the starch-containing material is preferably not less than 0.0001U, more preferably not less than 0.001U, particularly preferably not less than 0.01U, per 1 g of the starch-containing material, from the aspect of the level of the retrogradation suppressive effect. In this case, the amount of (D) to be added to the starch-containing material is preferably not more than 10U, more preferably not more than 1U, particularly preferably not more than 0.1U, per 1 g of the starch-containing material, from the aspect of preference for the properties.

In another embodiment, when the production method of the present invention includes adding (D) to a starch-containing material and, for example, glucoamylase or the like is used as (D), the amount of (D) to be added to the starch-containing material is preferably not less than 0.025U, more preferably not less than 0.25U, particularly preferably not less than 2.5U, per 1 g of the starch-containing material, from the aspect of the level of the retrogradation suppressive effect. In this case, the amount of (D) to be added to the starch-containing material is preferably not more than 2500U, more preferably not more than 250U, particularly preferably not more than 25U, per 1 g of the starch-containing material, from the aspect of preference for the properties.

In another embodiment, when the production method of the present invention includes adding (D) to a starch-containing material and, for example, $\alpha$-glucosidaseor the like is used as (D), the amount of (D) to be added to the starch-containing material is preferably not less than 0.00001U, more preferably not less than 0.0001U, particularly preferably not less than 0.001U, per 1 g of the starch-containing material, from the aspect of the level of the retrogradation suppressive effect. In this case, the amount of (D) to be added to the starch-containing material is preferably not more than 1U, more preferably not more than 0.1U, particularly preferably not more than 0.01U, per 1 g of the starch-containing material, from the aspect of preference for the properties.

In one embodiment, when the production method of the present invention includes adding (D) to a starch-containing material and, for example, $\alpha$-amylase or the like is used as (D), the activity ratio of the amount of (A) and the amount of (D) to be added to the starch-containing material is preferably (A):(D)=1:0.00001 - 10000, more preferably (A):(D)=1:0.0001 - 1000, particularly preferably (A):(D)=1:0.001 - 100, from the aspects of the level of the retrogradation suppressive effect and preference for the properties.

In another embodiment, when the production method of the present invention includes adding (D) to a starch-containing material and, for example, $\beta$-amylase or the like is used as (D), the activity ratio of the amount of (A) and the amount of (D) to be added to the starch-containing material is preferably (A):(D)=1:0.000001 - 1000, more preferably (A):(D)=1:0.00001 - 100, particularly preferably (A):(D)=1:0.0001 - 10, from the aspects of the level of the retrogradation suppressive effect and preference for the properties.

In another embodiment, when the production method of the present invention includes adding (D) to a starch-containing material and, for example, glucoamylase or the like is used as (D), the activity ratio of the amount of (A) and the amount of (D) to be added to the starch-containing material is preferably (A):(D)=1:0.00025 - 250000, more preferably (A):(D)=1:0.0025 - 25000, particularly preferably (A):(D)=1:0.025 - 2500, from the aspects of the level of the retrogradation suppressive effect and preference for the properties.

In another embodiment, when the production method of the present invention includes adding (D) to a starch-containing material and, for example, α-glucosidaseor the like is used as (D), the activity ratio of the amount of (A) and the amount of (D) to be added to the starch-containing material is preferably (A):(D)=1:0.0000001 - 100, more preferably (A):(D)=1:0.000001 - 10, particularly preferably (A):(D)=1:0.00001 - 1, from the aspects of the level of the retrogradation suppressive effect and preference for the properties.

When the production method of the present invention includes adding (E) to a starch-containing material, in addition to the addition of (A), and (C) or (D) to the starch-containing material, the amount of (E) to be added to the starch-containing material is preferably not less than 0.00001U, more preferably not less than 0.0001U, particularly preferably not less than 0.0005U, per 1 g of the starch-containing material, because the action of (A) can be effectively enhanced. In this case, the amount of (E) to be added to the starch-containing material is preferably not more than 10U, more preferably not more than 1U, further preferably not more than 0.1U, particularly preferably not more than 0.03U, per 1 g of the starch-containing material, because the action of (A) can be effectively enhanced.

In one embodiment, when the production method of the present invention further includes adding (E) to a starch-containing material, in addition to the addition of (A), and (C) or (D) to the starch-containing material and, for example, glucose oxidase or the like is used as (E), the amount of (E) to be added to the starch-containing material is preferably not less than 0.0001U, more preferably not less than 0.001U, particularly preferably not less than 0.002U, per 1 g of the starch-containing material, because the action of (A) can be effectively enhanced. In this case, the amount of (E) to be added to the starch-containing material is preferably not more than 1U, more preferably not more than 0.1U, particularly preferably not more than 0.05U, per 1 g of the starch-containing material, because the action of (A) can be effectively enhanced.

In another embodiment, when the production method of the present invention further includes adding (E) to a starch-containing material, in addition to the addition of (A), and (C) or (D) to the starch-containing material and, for example, transglutaminase or the like is used as (E), the amount of (E) to be added to the starch-containing material is preferably not less than 0.00001U, more preferably not less than 0.0001U, particularly preferably not less than 0.0005U, per 1 g of the starch-containing material, because the action of (A) can be effectively enhanced. In this case, the amount of (E) to be added to the starch-containing material is preferably not more than 1U, more preferably not more than 0.01U, particularly preferably not more than 0.005U, per 1 g of the starch-containing material, because the action of (A) can be effectively enhanced.

In one embodiment, when the production method of the present invention further includes adding (E) to a starch-containing material, in addition to the addition of (A), and (C) or (D) to the starch-containing material and, for example, glucose oxidase or the like is used as (E), the activity ratio of the amount of (A) and the amount of (E) to be added to the starch-containing material is preferably (A):(E)=1:0.0001 - 0.04, more preferably (A):(E)=1:0.0005 - 0.035, particularly preferably (A):(E)=1:0.001 - 0.03, because the action of (A) can be effectively enhanced.

In another embodiment, when the production method of the present invention further includes adding (E) to a starch-containing material, in addition to the addition of (A), and (C) or (D) to the starch-containing material and, for example, transglutaminase or the like is used as (E), the activity ratio of the amount of (A) and the amount of (E) to be added to the starch-containing material is preferably (A):(E)=1:0.00001 - 0.01, more preferably (A):(E)=1:0.0001 - 0.005, particularly preferably (A):(E)=1:0.00015 - 0.003, because the action of (A) can be effectively enhanced.

[Starch-containing material]

In the present invention, the "starch-containing material" refers to a material containing starch and used in the production of foods, and is a concept encompassing starch itself, processed starch and the like. Examples of the starch-containing material include materials obtained from plants containing starch as a component, and more specifically, materials and the like obtained by applying as necessary a processing treatment (e.g., grinding treatment, pulverization treatment, heat treatment, dry treatment, concentration treatment, etc.) to cereals (e.g., wheat, rice, corn, etc.), potatoes (e.g., potato, sweet potato, cassava, etc.), vegetables, fruits and the like. Specific examples include wheat flour (e.g., cake flour, all purpose flour, bread flour, etc.), rice, rice flour, whole corn, corn powder, corn paste, cut potatoes, potato flakes, potato powder, boiled potato and the like.

When the production method of the present invention includes adding (A) and (B) to a starch-containing material, (A) and (B) may be separately added to the starch-containing material, or (A) and (B) may be premixed before addition and the obtained mixture may be added. (A) and (B) may be added using the below-mentioned composition of the present invention.

When (A) and (B) are separately added, the order and interval of addition are not particularly limited and, for example, they may be added in the order of (A) and (B), or vice versa. In addition, (A) and (B) may be added simultaneously.

When the production method of the present invention includes adding (A) and (C) to a starch-containing material, (A) and (C) may be separately added to the starch-containing material, or (A) and (C) may be premixed before addition and the obtained mixture may be added. (A) and (C) may be added using the below-mentioned composition of the present invention.

When (A) and (C) are separately added, the order and interval of addition are not particularly limited and, for example, they may be added in the order of (A) and (C), or vice versa. In addition, (A) and (C) may be added simultaneously.

When the production method of the present invention includes adding (A) and (D) to a starch-containing material, (A) and (D) may be separately added to the starch-containing material, or (A) and (D) may be premixed before addition and the obtained mixture may be added. (A) and (D) may be added using the below-mentioned composition of the present invention.

When (A) and (D) are separately added, the order and interval of addition are not particularly limited and, for example, they may be added in the order of (A) and (D), or vice versa. In addition, (A) and (D) may be added simultaneously.

When the production method of the present invention further includes adding (E) to a starch-containing material, in addition to the addition of (A), and (C) or (D) to the starch-containing material, (A) and (C) - (E) may be separately added to the starch-containing material, or (A) and (C) - (E) may be premixed before addition and the obtained mixture may be added. (A) and (C) - (E) may be added using the below-mentioned composition of the present invention.

When (A) and (C) - (E) are separately added, the order and interval of addition are not particularly limited and, for example, they may be added in the order of (A), (C) or (D), (E), or vice versa. In addition, (A) and (C) - (E) may be added simultaneously.

The method and conditions for adding (A) - (E) to a starch-containing material are not particularly limited, and can be appropriately set according to the kind and the like of the starch-containing food. The timing of adding (A) - (E) to the starch-containing material is not particularly limited. For example, when the production method of the present invention includes heating the starch-containing material, the addition of (A) - (E) is preferably performed before heating the starch-containing material.

The production method of the present invention may appropriately include, in addition to adding (A) - (E) to a starch-containing material, processing steps and cooking steps conventionally used in the production of starch-containing foods, according to the kind and the like of the starch-containing food to be produced. For example, the production method of the present invention may further include heating the starch-containing material.

When the production method of the present invention includes heating a starch-containing material, the method and conditions for heating (e.g., heating temperature, heating time, etc.) the starch-containing material are not particularly limited, and can be appropriately set according to the kind and the like of the starch-containing food to be produced. The heating temperature in heating a starch-containing material is generally 60 - 250°C, and the heating time is generally 1 - 180 min.

In the production method of the present invention, the starch-containing food may be subjected to a refrigeration treatment, a freezing treatment, or the like, depending on the desired distribution form of the starch-containing food. Therefore, the starch-containing food obtained by the production method of the present invention may be a refrigerated product, a frozen product (frozen food), or the like.

In the production method of the present invention, food materials other than (A) - (E) and starch-containing materials may be appropriately used depending on the kind and the like of the starch-containing food to be produced.

While the kind of the starch-containing food that can be produced by the production method of the present invention is not particularly limited, the production method of the present invention is preferably a method for producing a starch-containing food selected from the group consisting of bakery food (e.g., bread, cake, cookie, etc.), rice food (e.g., cooked rice, fried rice, rice ball, etc.), potato food (e.g., mashed potato, french fries, potato salad, potato flakes, etc.), dough sheet food (e.g., Japanese wheat noodles, Chinese noodles, gyoza, etc.), and rice-cakes (e.g., rice-cake, rice-flour dumpling, arrowroot-starch cake, etc.) (more preferably, the group consisting of bakery food, rice food, potato food and dough sheet food).

In one embodiment, the production method of the present invention is preferably a method for producing a bakery food, more preferably bread.

In another embodiment, the production method of the present invention is preferably a method for producing a potato food.

In another embodiment, the production method of the present invention is preferably a method for producing rice-cakes.

According to According to the production method of the present invention, a modified starch-containing food can be produced. Specifically, for example, a starch-containing food with suppressed retrogradation, a starch-containing food with improved texture, a starch-containing food with improved production suitability, a starch-containing food with improved flavor, a starch-containing food with modified property and the like can be produced.

According to According to the production method of the present invention, a starch-containing food with suppressed retrogradation can be produced.

In the present invention, "retrogradation" of a starch-containing food" means that the quality of the starch-containing food deteriorates with time (for example, the texture of the starch-containing food becomes hard, becomes dry and crumbly, dry feeling becomes stronger over time, and the like). The degree of retrogradation of starch-containing foods can be evaluated by a sensory evaluation by an expert panel (e.g., sensory evaluation shown in Examples described later, etc.).

In the present invention, the "suppression" of retrogradation of starch-containing foods means preventing or retarding retrogradation of the starch-containing foods.

According to the production method of the present invention, a starch-containing food with improved texture can also be produced. For example, a starch-containing food improved in at least one selected from the group consisting of crispness, meltability in a mouth, moist feeling, easiness in loosening, resilience, softness, balance between softness and firmness in grain and smoothness, and the like can be produced.

In one embodiment, when the starch-containing food is a bakery food, for example, a bakery food with improved crispness, a bakery food with improved meltability in a mouth, a bakery food with improved moist feeling, a bakery food with improved softness, and the like can be produced according to the production method of the present invention. In the present invention, the "crispness" of a bakery food means the ease of biting off when the food is chewed with the front tooth. The "meltability in a mouth" of a bakery food means the ease of melting of a lump of food in the mouth during chewing and, for example, a food that easily forms a lump and remains in the mouth tends to be evaluated as having poor meltability in the mouth. The "moist feeling" of a bakery food means a texture affording a feeling of the presence of an appropriate amount of water and free of dryness. The degree of crispness, meltability in a mouth, and moist feeling of a bakery food can be evaluated, for example, by a sensory evaluation by an expert panel (e.g., sensory evaluation shown in Examples described later, etc.) and the like.

In another embodiment, when the starch-containing food is a rice food, for example, a rice food with improved easiness in loosening, a rice food with improved resilience, a rice food with an improved balance between softness and firmness in grain and the like can be produced according to the production method of the present invention.

In another embodiment, when the starch-containing food is a potato food, for example, a potato food with improved smoothness by suppressing dryness and crumbly feeling, a potato food with improved moist feeling, a potato food with improved softness and the like can be produced according to the production method of the present invention.

According to the production method of the present invention, a starch-containing food with improved production suitability can also be produced. In the present invention, the "production suitability" of a starch-containing food means ease of handling in producing a starch-containing food.

In one embodiment, when the starch-containing food is cooked rice which is one kind of rice food, for example, cooked rice suppressed in adhered residue to a rice cooking pot and the like can be produced according to the production method of the present invention.

According to the production method of the present invention, a starch-containing food with improved flavor can be produced. In the present invention, the "flavor" of the starch-containing food means the aroma, taste, or their overall sensation of the starch-containing food.

In one embodiment, when the starch-containing food is a potato food, for example, a potato food with an improved potato feeling (aroma, flavor unique to steamed potatoes) and the like can be produced according to the production method of the present invention. The presence or absence and the degree of potato feeling of the potato food can be evaluated, for example, by a sensory evaluation by an expert panel (e.g., sensory evaluation shown in Examples described later, etc.).

According to the production method of the present invention, preferably, a starch-containing food with suppressed retrogradation and improved texture can be produced.

According to the production method of the present invention, preferably, a starch-containing food with improved production suitability and improved texture can be produced.

According to the production method of the present invention, preferably, a starch-containing food with suppressed retrogradation, or improved production suitability, and improved texture can be produced.

According to the production method of the present invention, preferably, a starch-containing food with improved texture and flavor can be produced.

According to the production method of the present invention, a starch-containing food with modified property can also be produced.

In one embodiment, when the starch-containing food is a bakery food, for example, a bakery food with improve swelling during baking (bake elongation), and the like can be produced according to the production method of the present invention. The degree of swelling of the bakery food during baking can be evaluated, for example, by the size (e.g., height, etc.) of the bakery food.

2. Method for modifying starch-containing food

The present invention also provides a method for modifying a starch-containing food, comprising (i) adding (A) an amylomaltase derived from a bacterium of the genus Thermus to the starch-containing material, and (ii) further adding (B) a protein or lipid modification enzyme, or (C) a starch degradation product or (D) a starch degradation enzyme to the starch-containing material (sometimes to be referred to as "the modification method of the present invention" in the present specification). The modification method of the present invention may be, for example, a method for suppressing retrogradation of a starch-containing food, a method for improving texture of a starch-containing food, a method for improving production suitability of a starch-containing food, a method for improving a flavor of a starch-containing food, a method for modifying property of a starch-containing food or the like.

In one embodiment, the modification method of the present invention may be a method for suppressing retrogradation of a starch-containing food, and therefore, the present invention also provides a method for suppressing retrogradation of a starch-containing food, comprising (i) adding (A) an amylomaltase derived from a bacterium of the genus Thermus to the starch-containing material, and (ii) further adding (B) a protein or lipid modification enzyme, or (C) a starch degradation product or (D) a starch degradation enzyme to the starch-containing material.

In another embodiment, the modification method of the present invention may be a method for improving texture of a starch-containing food, and therefore, the present invention also provides a method for improving texture of a starch-containing food, comprising (i) adding (A) an amylomaltase derived from a bacterium of the genus Thermus to the starch-containing material, and (ii) further adding (B) a protein or lipid modification enzyme, or (C) a starch degradation product or (D) a starch degradation enzyme to the starch-containing material (preferably, a method for improving at least one selected from the group consisting of crispness, meltability in a mouth, resilience, balance between softness and firmness in grain, and smoothness of a starch-containing food).

In another embodiment, the modification method of the present invention may be a method for improving production suitability of a starch-containing food, and therefore, the present invention also provides a method for improving production suitability of a starch-containing food, comprising adding (A) an amylomaltase derived from a bacterium of the genus Thermus, and (B) a protein or lipid modification enzyme to the starch-containing material.

In another embodiment, the modification method of the present invention may be a method for improving a flavor of a starch-containing food, and therefore, the present invention also provides a method for improving a flavor of a starch-containing food, comprising adding (A) an amylomaltase derived from a bacterium of the genus Thermus, and (B) a protein or lipid modification enzyme to the starch-containing material.

In another embodiment, the modification method of the present invention may be a method for modifying property of a starch-containing food, and therefore, the present invention also provides a method for modifying property of a starch-containing food, comprising (i) adding (A) an amylomaltase derived from a bacterium of the genus Thermus to the starch-containing material, and (ii) further adding (B) a protein or lipid modification enzyme, or (C) a starch degradation product or (D) a starch degradation enzyme to the starch-containing material.

The modification method of the present invention may be a method for suppressing retrogradation and improving texture of a starch-containing food. In addition, the modification method of the present invention may be a improving production suitability and texture of a starch-containing food. The modification method of the present invention may be a improving a flavor and texture of a starch-containing food.

The (A) (amylomaltase derived from bacterium of the genus Thermus), (B) (protein or lipid modification enzyme) and the starch-containing material used in the modification method of the present invention are the same as (A), (B) and the starch-containing material explained in the aforementioned "1. Production method of starch-containing food", and preferred embodiments are also the same.

The (C) (starch degradation product) that may be used in the modification method of the present invention is the same as (C) explained in the aforementioned "1. Production method of starch-containing food", and preferred embodiments are also the same.

The (D) (starch degradation enzyme) that may be used in the modification method of the present invention is the same as (D) explained in the aforementioned "1. Production method of starch-containing food", and preferred embodiments are also the same.

The modification method of the present invention may further include adding (E) a protein modification enzyme to a starch-containing material, in addition to the addition of (A), and (C) or (D) to the starch-containing material.

The (E) (protein modification enzyme) that may be used in the method of the present invention is the same as (E) explained in the aforementioned "1. Production method of starch-containing food", and preferred embodiments are also the same.

Unless otherwise specified, the modification method of the present invention can be performed in the same manner as in the production method of the present invention, and preferred embodiments are also the same.

While the kind of the starch-containing food for which the modification method of the present invention is used is not particularly limited, the modification method of the present invention is preferably a method for suppressing retro-

gradation of a starch-containing food selected from the group consisting of bakery food (e.g., bread, cake, cookie, etc.), rice food (e.g., cooked rice, fried rice, rice ball, etc.), potato food (e.g., mashed potato, french fries, potato salad, potato flakes, etc.), dough sheet food (e.g., Japanese wheat noodles, Chinese noodles, gyoza, etc.), and rice-cakes (e.g., rice-cake, rice-flour dumpling, arrowroot-starch cake, etc.) (more preferably, the group consisting of bakery food, rice food, potato food and dough sheet food).

In one embodiment, the modification method of the present invention is preferably a method for suppressing retrogradation of bakery food, a method for improving texture of bakery food, more preferably, a method for suppressing retrogradation of bread, a method for improving texture of bread.

In another embodiment, the modification method of the present invention is preferably a method for suppressing retrogradation of potato food.

In another embodiment, the modification method of the present invention is preferably a method for improving production suitability of rice food, a method for improving texture of rice food, more preferably, a method for improving production suitability of cooked rice, a method for improving texture of cooked rice.

In another embodiment, the modification method of the present invention is preferably a method for improving texture and a flavor of potato food, more preferably, a method for improving texture and a flavor of mashed potato.

According to the modification method of the present invention, retrogradation of a starch-containing food can be suppressed. For example, hardened texture of a starch-containing food over time, dry and crumbly texture of a starch-containing food over time, stronger dry feeling of the texture of a starch-containing food over time, and the like can be suppressed according to the modification method of the present invention.

According to the modification method of the present invention, texture of a starch-containing food can be improved and, for example, at least one selected from the group consisting of crispness, meltability in a mouth, moist feeling, easiness in loosening, resilience, softness, balance between softness and firmness in grain and smoothness of a starch-containing food can be improved.

In one embodiment, when the starch-containing food is a bakery food, for example, crispness, meltability in a mouth, moist feeling, softness and the like of the bakery food can be improved according to the modification method of the present invention.

In another embodiment, when the starch-containing food is a rice food, for example, easiness in loosening, resilience, balance between softness and firmness in grain and the like of the rice food can be improved according to the modification method of the present invention.

In another embodiment, when the starch-containing food is a potato food, for example, smoothness and the like can be improved by suppressing dry and crumbly feeling of the potato food according to the modification method of the present invention. In addition, moist feeling, softness and the like of potato foods can be improved.

According to the modification method of the present invention, production suitability of a starch-containing food can be improved.

In one embodiment, when the starch-containing food is cooked rice which is one kind of rice food, for example, adhered residue of cooked rice to a rice cooking pot and the like can be improved according to the modification method of the present invention.

According to the modification method of the present invention, a flavor of a starch-containing food can be improved.

In one embodiment, when the starch-containing food is a potato food, for example, potato feeling and the like of the potato food can be improved according to the modification method of the present invention.

According to the modification method of the present invention, the property of a starch-containing food can also be modified.

In one embodiment, when the starch-containing food is a bakery food, for example, swelling of bakery food during baking (bake elongation) and the like can be improved according to the method of the present invention.


3. Enzyme composition

The present invention also provides an enzyme composition comprising (i) (A) an amylomaltase derived from a bacterium of the genus Thermus, and
(ii) (B) a protein or lipid modification enzyme, or (C) a starch degradation product or (D) a starch degradation enzyme (sometimes to be referred to as "the enzyme composition of the present invention" in the present specification).

The (A) (amylomaltase derived from bacterium of the genus Thermus) and (B) (protein or lipid modification enzyme) contained in the enzyme composition of the present invention are the same as (A) and (B) explained in the aforementioned "1. Production method of starch-containing food", and preferred embodiments are also the same.

The (C) (starch degradation product) that may be contained in that may be contained in the enzyme composition of the present invention is the same as (C) explained in the aforementioned "1. Production method of starch-containing food", and preferred embodiments are also the same.

The (D) (starch degradation enzyme) that may be contained in the enzyme composition of the present invention is the same as (D) explained in the aforementioned "1. Production method of starch-containing food", and preferred embodiments are also the same.

The enzyme composition of the present invention may further contain (E) a protein modification enzyme in addition to (A), and (C) or (D).

The (E) (protein modification enzyme) that may be contained in the enzyme composition of the present invention is the same as (E) explained in the aforementioned "1. Production method of starch-containing food", and preferred embodiments are also the same.

While the amount of (A) contained in the enzyme composition of the present invention is not particularly limited, it is generally 0.00001 - 10000U, preferably 0.0001 - 1000U, per 1 g of the enzyme composition of the present invention.

When the enzyme composition of the present invention contains (B), the amount of (B) contained in the enzyme composition of the present invention is not particularly limited, and is generally 0.00001 - 10000U, preferably 0.0001 - 1000U, per 1 g of the enzyme composition of the present invention.

When the enzyme composition of the present invention contains (C), the amount of (C) contained in the enzyme composition of the present invention is not particularly limited, and is generally 0.1 - 99 wt%, preferably 1 - 90 wt%, of the enzyme composition of the present invention.

When the enzyme composition of the present invention contains (D), the amount of (D) contained in the enzyme composition of the present invention is not particularly limited, and is generally 0.00001 - 10000U, preferably 0.0001 - 1000U, per 1 g of the enzyme composition of the present invention.

When the enzyme composition of the present invention contains (E), the amount of (D) contained in the enzyme composition of the present invention is not particularly limited, and is generally 0.00001 - 10000U, preferably 0.0001 - 1000U, per 1 g of the enzyme composition of the present invention.

When the enzyme composition of the present invention contains (B), the activity ratio of the content of (A) and the content of (B) in the enzyme composition of the present invention is the same as the activity ratio of the amount of (A) and the amount of (B) to be added to the starch-containing material explained in the aforementioned "1. Production method of starch-containing food", and preferred range is also the same.

When the enzyme composition of the present invention contains (D), the activity ratio of the content of (A) and the content of (D) in the enzyme composition of the present invention is the same as the activity ratio of the amount of (A) and the amount of (D) to be added to the starch-containing material explained in the aforementioned "1. Production method of starch-containing food", and preferred range is also the same.

When the enzyme composition of the present invention contains (E), the activity ratio of the content of (A) and the content of (E) in the enzyme composition of the present invention is the same as the activity ratio of the amount of (A) and the amount of (E) to be added to the starch-containing material explained in the aforementioned "1. Production method of starch-containing food", and preferred range is also the same.

While the form of the enzyme composition of the present invention is not particularly limited, for example, solid (including powder, granular and the like), liquid (including slurry and the like), gel, paste and the like can be mentioned.

The enzyme composition of the present invention may consist only of (A) - (E). In addition to these, it may further contain a base conventionally used for an enzyme preparation for foods. Examples of the base include starch, dextrin, cyclodextrin, saccharides (e.g., lactose, sucrose, glucose, etc.), proteins (e.g., animal and plant proteins, etc.), salts (sodium chloride, etc.), water, fats and oils, and the like.

The enzyme composition of the present invention may further contain, for example, excipient, pH adjuster, antioxidant, thickening stabilizer, emulsifier, sweetener (e.g., sugar, etc.), cooking salt, organic salts, inorganic salts, seasoning, acidulant, spice, colorant, color former, and the like in addition to (A) - (E), as long as the purpose of the present invention is not impaired.

The enzyme composition of the present invention can be produced according to a method conventionally used for producing an enzyme preparation for food or a method analogous thereto.

All of (A) - (E) to be contained in the enzyme composition of the present invention may be contained in one preparation, or (A) - (E) may be contained in two or more preparations. When (A) - (E) are contained in two or more preparations, the enzyme composition of the present invention may be, for example, a combination of preparations separately containing each of (A) - (E), or the like.

When (A) - (E) to be contained in the enzyme composition of the present invention are contained in two or more preparations, the amount of (A) - (E) contained in the enzyme composition of the present invention is calculated by totaling the amounts of (A) - (E) contained in each preparation.

The enzyme composition of the present invention may be used by adding to a starch-containing material which is a material for starch-containing foods, and the enzyme composition of the present invention may be an enzyme composition for starch-containing foods.

The starch-containing material that may be added to the enzyme composition of the present invention is the same as the starch-containing material explained in the aforementioned "1. Production method of starch-containing food", and preferred embodiments are also the same.

The method and conditions for adding the enzyme composition of the present invention to a starch-containing material are not particularly limited, and can be appropriately performed by a method known per se or a method analogous thereto according to the form of the enzyme composition of the present invention and the kind of the starch-containing food and the like. The timing of adding the enzyme composition of the present invention to the starch-containing material is not particularly limited. For example, when a starch-containing food is produced by heating a starch-containing material, the addition of the enzyme composition of the present invention is preferably performed before heating the starch-containing material.

The enzyme composition of the present invention may be added to a starch-containing material such that the amount of (A) added to the starch-containing material is the same as the amount described in the aforementioned "1. Production method of starch-containing food".

When the enzyme composition of the present invention contains (B), the enzyme composition of the present invention may be added to a starch-containing material such that the amount of (B) added to the starch-containing material is the same as the amount described in the aforementioned "1. Production method of starch-containing food".

When the enzyme composition of the present invention contains (C), the enzyme composition of the present invention may be added to a starch-containing material such that the amount of (C) added to the starch-containing material is the same as the amount described in the aforementioned "1. Production method of starch-containing food".

When the enzyme composition of the present invention contains (D), the enzyme composition of the present invention may be added to a starch-containing material such that the amount of (D) added to the starch-containing material is the same as the amount described in the aforementioned "1. Production method of starch-containing food".

When the enzyme composition of the present invention contains (E), the enzyme composition of the present invention may be added to a starch-containing material such that the amount of (E) added to the starch-containing material is the same as the amount described in the aforementioned "1. Production method of starch-containing food".

The enzyme composition of the present invention may be preferably used as an enzyme composition for starch-containing foods. While the kind of the starch-containing food for which the enzyme composition of the present invention is used is not particularly limited, the enzyme composition of the present invention is preferably an enzyme composition for a starch-containing food selected from the group consisting of bakery food (e.g., bread, cake, cookie, etc.), rice food (e.g., cooked rice, fried rice, rice ball, etc.), potato food (e.g., mashed potato, french fries, potato salad, potato flakes, etc.), dough sheet food (e.g., Japanese wheat noodles, Chinese noodles, gyoza, etc.), and rice-cakes (e.g., rice-cake, rice-flour dumpling, arrowroot-starch cake, etc.) (more preferably, the group consisting of bakery food, rice food, potato food and dough sheet food).

The enzyme composition of the present invention may be preferably used as an enzyme composition for modifying starch-containing foods. The enzyme composition for modifying starch-containing foods may be, for example, an enzyme composition for suppressing retrogradation of starch-containing foods, an enzyme composition for improving texture of starch-containing foods, an enzyme composition for improving production suitability of starch-containing foods, an enzyme composition for improving flavor of starch-containing foods, an enzyme composition for modifying property of starch-containing foods, or the like.

While the kind of the starch-containing food for which the enzyme composition of the present invention is used is not particularly limited, the enzyme composition of the present invention is preferably an enzyme composition for suppressing retrogradation of a starch-containing food selected from the group consisting of bakery food (e.g., bread, cake, cookie, etc.), rice food (e.g., cooked rice, fried rice, rice ball, etc.), potato food (e.g., mashed potato, french fries, potato salad, potato flakes, etc.), dough sheet food (e.g., Japanese wheat noodles, Chinese noodles, gyoza, etc.), and rice-cakes (e.g., rice-cake, rice-flour dumpling, arrowroot-starch cake, etc.) (more preferably, the group consisting of bakery food, rice food, potato food and dough sheet food).

In one embodiment, the enzyme composition of the present invention is preferably an enzyme composition for suppressing retrogradation of bakery foods, more preferably, an enzyme composition for suppressing retrogradation of bread.

In another embodiment, the enzyme composition of the present invention is preferably an enzyme composition for suppressing retrogradation of potato foods.

In another embodiment, the enzyme composition of the present invention is preferably an enzyme composition for improving texture of bakery foods, more preferably, an enzyme composition for improving texture of bread.

In another embodiment, the enzyme composition of the present invention is preferably an enzyme composition for improving production suitability of rice foods, more preferably, an enzyme composition for improving production suitability of cooked rice.

In another embodiment, the enzyme composition of the present invention is preferably an enzyme composition for improving texture and flavor of potato foods, more preferably, an enzyme composition for improving texture and flavor of mashed potato.

The enzyme composition of the present invention may be preferably used as an enzyme composition for suppressing retrogradation of starch-containing foods. The enzyme composition of the present invention may be preferably used for suppressing, for example, hardened texture of starch-containing foods over time, dry and crumbly texture of starch-containing foods over time, stronger dry feeling of the texture of starch-containing foods over time, and the like.

The enzyme composition of the present invention may also be used preferably as an enzyme composition for improving the texture of starch-containing foods. For example, it may be used as an enzyme composition for improving at least one selected from the group consisting of crispness, meltability in a mouth, moist feeling, easiness in loosening, resilience, softness, balance between softness and firmness in grain and smoothness of starch-containing foods.

In one embodiment, when the starch-containing food is a bakery food, the enzyme composition of the present invention may be preferably used, for example, as an enzyme composition for improving crispness, meltability in a mouth, moist feeling, softness and the like of bakery foods.

In another embodiment, when the starch-containing food is a rice food, the enzyme composition of the present invention may be preferably used, for example, as an enzyme composition for improving easiness in loosening, resilience, balance between softness and firmness in grain and the like of rice foods.

In another embodiment, when the starch-containing food is a potato food, the enzyme composition of the present invention may be preferably used, for example, as an enzyme composition for improving smoothness and the like by suppressing dry and crumbly feeling of potato foods. Also, the enzyme composition of the present invention may also be used preferably, for example, as an enzyme composition for improving moist feeling, softness and the like of potato foods.

The enzyme composition of the present invention may also be used preferably as an enzyme composition for improving production suitability of starch-containing foods.

In one embodiment, when the starch-containing food is cooked rice which is one kind of rice food, the enzyme composition of the present invention may be preferably used, for example, as an enzyme composition for suppressing adhered residue of cooked rice to a rice cooking pot.

The enzyme composition of the present invention may also be used as an enzyme composition for improving production suitability and texture of starch-containing foods. In addition, the enzyme composition of the present invention may also be used as an enzyme composition for suppressing retrogradation and improving texture of starch-containing foods.

The enzyme composition of the present invention may also be used preferably as an enzyme composition for improving a flavor of starch-containing foods.

In one embodiment, when the starch-containing food is a potato food, the enzyme composition of the present invention may be preferably used, for example, as an enzyme composition for improving potato feeling.

The enzyme composition of the present invention may also be used as an enzyme composition for improving texture and flavor of starch-containing foods.

The enzyme composition of the present invention may also be used as an enzyme composition for modifying property of starch-containing foods.

In one embodiment, when the starch-containing food is a bakery food, the enzyme composition of the present invention may be preferably used, for example, as an enzyme composition for improving swelling of bakery foods during baking (bake elongation) and the like.

The present invention is more specifically explained in the following Examples; however, the present invention is not limited at all by the Examples.

[Example]

The Thermus thermophilus-derived amylomaltase aqueous solution used in the following Experimental Examples was prepared by the following method.

1. Production of expression vector

Unless otherwise specified, the restriction enzymes used were manufactured by Takara Bio Inc., and the other reagents used were manufactured by Wako Pure Chemical Industries, Ltd. Further, Thermus thermophilus-derived amylomaltase is abbreviated as "TtAM" below. The expression vector of TtAM was prepared as follows. Based on the amino acid sequence of accession number YP_144527, a DNA having a sequence optimized for Escherichia coli codon usage was synthesized by Genscript. An NdeI restriction enzyme sequence was added to the 5'-terminal of the synthetic DNA, and an EcoRI restriction enzyme sequence was added to the 3'-terminal. After treatment with both restriction enzymes, the DNA was inserted into an expression vector pET21a(+) treated with restriction enzymes NdeI and RcoI to obtain a TtAM expression vector pET21a-TtAM.

## 2. Production of expression bacteria

Using the obtained expression vector, Escherichia coli BL21(DE3) was transformed to obtain Escherichia coli expression strain BL21(DE3)/pET21a-TtAM.

## 3. Mass expression, purification

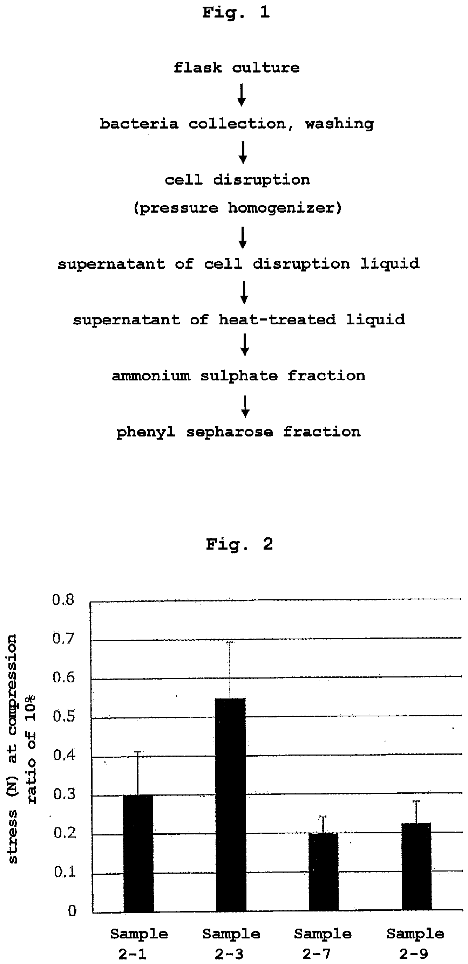

The purification flow is shown in Fig. 1. The produced TtAM expression bacterium (BL21(DE3)/pET21a-TtAM) was cultured in 24 L of an expression medium (1% tryptone (Nihon Pharmaceutical Co., Ltd.), 0.5% yeast extract (Kyokuto Pharmaceutical Industrial Co., Ltd.), 0.5% sodium chloride, 0.5% Hicasamino Acids "DAIGO" (Nihon Pharmaceutical Co., Ltd.), 0.2% glucose, 50 μg/mL ampicillin) for 30°C. IPTG (isopropyl-β-thiogalactopyranoside) was added around OD600=0.6 to a final concentration of 20 μM, and culture was continued at 30°C for 20 hr. The cells were collected, and the obtained bacterial cells (219 g) were suspended in about 440 mL of 20 mM Tris-HCl (pH 8.0) and 0.15 M NaCl, and disrupted by a pressure type homogenizer (manufactured by SMT Co., Ltd., pressure type homogenizer LAB1000). For disruption, an ultrasonic disintegrator (manufactured by Tomy Seiko Co., Ltd., ultrasonic disintegrator UD-201, standard chip TP-012 (φ14.5 mm)) was used in combination. The cell homogenate was centrifuged at 22,000×g for 15 min and the cell extract supernatant (about 600 mL) was obtained. The cell extract supernatant was heat-treated at 70°C for 30 min, and centrifuged at 22,000xg for 20 min. Ammonium sulfate was added to about 465 mL of the collected supernatant to 50% saturation, the supernatant was allowed to stand overnight at 4°C, and the protein was precipitated by centrifugation at 22,000×g for 20 min, a small amount thereof was suspended in 20 mM Tris-HCl (pH 7.5) and dialyzed against 20 mM Tris-HCl (pH 7.5). The protein solution after dialysis was about 120 mL.

Then, ammonium sulfate was added to the protein solution to 20% saturation, the mixture was centrifuged at 22,000×g for 15 min and the supernatant was recovered. The supernatant was subjected to a 0.45 μm filter treatment after which to hydrophobic chromatography in two portions using Phenyl Sepharose 6 Fast Flow (high sub) 50/10 column (column volume 200 mL, GE healthcare). The column was washed with 20 mM Tris-HCl (pH 7.5), 20% saturated ammonium sulfate. After a linear concentration gradient of 20 mM Tris-HCl (pH 7.5) and saturated ammonium sulfate of 20% to 0%, the enzyme of interest was eluted with ultrapure water (Milli Q water). The obtained TtAM elution fraction was collected, and finally dialyzed against 20 mM potassium phosphate buffer (pH 7.0) and 25 mM NaCl, and then sterilized by a 0.22 μm filter to obtain a TtAM purified product (2730U/mL).

<Experimental Example 1>

[Production of white bread]

(Sample 1-1)

Bread flour (manufactured by Nippon Flour Mills Co., Ltd., trade name "NIPPN EAGLE (bread flour)"), granulated sugar (manufactured by Mitsui Sugar Co., Ltd., trade name "SPOON brand Granulated sugar"), cooking salt (manufactured by Naikai Salt Industries Co., LTD., trade name "Nakuru M"), and skim milk (manufactured by MORINAGA MILK INDUSTRY CO., LTD., trade name "MORINAGA SKIM MILK") were premixed in the amounts shown in Table 1 to give a mixed powder.

After weighing 184.8 g of city water in a container (bread case with blades) of a commercially available automatic home bakery (automatic bread-baking machine for home use) (manufactured by MK Seiko Co., Ltd., product number: HBK-100), the above-mentioned mixed powder was added, 3 g of dry yeast (manufactured by Nisshin Foods Inc., trade name "Nissin Super Kameriya Dry Yeast") and 14 g of shortening (manufactured by J-OIL MILLS, Inc., trade name "FASHIE") were added on the mixed powder so as not to touch the city water. The menu of the automatic home bakery was set to "white bread/baked color: normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (20°C) for 1 hr.

[Table 1]

| material | weight (g) |
| --- | --- |
| bread flour | 280 |
| granulated sugar | 22.4 |
| cooking salt | 4.2 |

(continued)

| material | weight (g) |
|---|---|
| skim milk | 5.6 |

(Sample 1-2)

By the same procedure as in Sample 1-1 except that 0.024U of glucose oxidase (manufactured by Nippon Chemical Industrial Co., Ltd., trade name "Sumiteam PGO") was also added per 1 g of bread flour when a mixed powder was put into the container of a commercially available automatic home bakery, white bread was produced.

(Sample 1-3)

By the same procedure as in Sample 1-1 except that 1.0U of Thermus thermophilus-derived amylomaltase aqueous solution was added per 1 g of bread flour before a mixed flour was put into a container of a commercially available automatic home bakery, white bread was produced.

(Sample 1-4)

By the same procedure as in Sample 1-3 except that 5.0U of Thermus thermophilus-derived amylomaltase aqueous solution was added per 1 g of bread flour, white bread was produced.

(Sample 1-5)

By the same procedure as in Sample 1-3 except that 0.024U of glucose oxidase (manufactured by Nippon Chemical Industrial Co., Ltd., trade name "Sumiteam PGO") was also added per 1 g of bread flour when a mixed powder was put into the container of a commercially available automatic home bakery, white bread was produced.

(Sample 1-6)

By the same procedure as in Sample 1-5 except that 5.0U of Thermus thermophilus-derived amylomaltase aqueous solution was added per 1 g of bread flour, white bread was produced.

(Measurement of bread height and calculation of bake elongation)

The height of the central portion (also referred to as "bread height" in the present specification) was measured for each of the white breads of Samples 1-1 to 1-6 after allowing to cool at room temperature (20°C) for 1 hr.
From the measured bread height, the bake elongation (%) of the breads of Samples 1-2 to 1-6 was calculated based on the following formula:

$$[\text{Bake elongation (\%)}] = [\text{Bread height (cm)}] \div [\text{Bread height (cm) of Sample 1-1}] \times 100$$

(Sensory evaluation)

After allowing to cool at room temperature (20°C) for 1 hr, the white breads of Samples 1-1 to 1-6 were sliced to a thickness of 2 cm, tightly sealed in a polyethylene bag, and stored at temperature of 10°C, humidity 50%. After storage for 2 days, an evaluation panel of 4 people ate only the crumb part (internal phase part of white bread) of each white bread, and retrogradation feeling, crispness, meltability in a mouth and preference were each evaluated based on the following criteria. The sensory evaluation was performed by a disgorging method.

[Retrogradation feeling]

++++: stronger retrogradation feeling than Sample 1-1

+++: feeling of retrogradation equivalent to Sample 1-1

++: less retrogradation feeling than Sample 1-1

+: clearly less retrogradation feeling than Sample 1-1

±: very low retrogradation feeling compared to Sample 1-1

-: no retrogradation feeling

As used herein, the "retrogradation feeling" refers to a sensation (hard texture, dry feeling, etc.) felt when eating a retrograded starch-containing food.

[Crispness]

++: very good crispness compared to white bread of Sample 1-1

+: good crispness compared to white bread of Sample 1-1

±: crispness equivalent to that of white bread of Sample 1-1

-: poor crispness compared to white bread of Sample 1-1

[Meltability in mouth]

++: very good meltability in mouth compared to white bread of Sample 1-1

+: good meltability in mouth compared to white bread of Sample 1-1

±: meltability in mouth equivalent to that of white bread of Sample 1-1

-: poor meltability in mouth compared to white bread of Sample 1-1

[Preference]

⊙: extremely preferable

O: very preferable

Δ: preferable

×: not preferable

The results are shown in the following Table 2. In the Table, "GO" is glucose oxidase and "TtAM" is thermus thermophilus-derived amylomaltase.

[Table 2]

| sample | 1-1 | 1-2 | 1-3 | 1-4 | 1-5 | 1-6 |
|---|---|---|---|---|---|---|
| GO (unit per 1 g of bread flour) | | 0.024 | | | 0.024 | 0.024 |
| TtAM (unit per 1 g of bread flour) | | | 1.0 | 5.0 | 1.0 | 5.0 |
| bread height (cm) | 14.2 | 14.3 | 15.0 | 15.3 | 15.0 | 15.2 |
| bake elongation (%) | | 100.7 | 105.6 | 107.7 | 105.6 | 107.0 |

(continued)

| sample | 1-1 | 1-2 | 1-3 | 1-4 | 1-5 | 1-6 |
|---|---|---|---|---|---|---|
| | +++ | +++ | ++ | + | ± | ± to - |
| retrogradation feeling | texture was hard, strong dry feeling was felt | dry feeling equivalent to Sample 1-1 was felt | dry feeling was suppressed than Sample 1-1 | clearly soft texture, slight dry feeling was felt | soft feeling almost equivalent to immediately after baking in Sample 1-1 was felt, moist feeling was higher than in Sample 1-3 | soft feeling almost equivalent to immediately after baking in Sample 1-1 was felt, moist feeling was further stronger than in Sample 1-5 |
| crispness | ± ( standard ) | ± | ± | - | + | + |
| meltability in mouth | ± ( standard ) | ± | ± | - | + | + |
| preference | × | × | Δ | O | ⊙ | ⊙ |

As is clear from the results shown in Table 2, a strong retrogradation suppressive effect was confirmed in the white bread of Sample 1-5, and a moist feeling was further improved in the white bread of Sample 1-6 and texture of almost freshly baked bread was obtained. Both the white breads of Sample 1-5 and Sample 1-6 showed good crispness and good meltability in mouth.

On the other hand, a clear retrogradation suppressive effect was not observed in the white bread of Sample 1-2 to which glucose oxidase was added alone.

<Experimental Example 2>

[Production of white bread]

(Sample 2-1)

After weighing 184.8 g of city water in a container (bread case with blades) of a commercially available automatic home bakery (automatic bread-baking machine for home use) (manufactured by MK Seiko Co., Ltd., product number: HBK-100), a mixed powder (312.2 g) prepared in the same manner as in Experimental Example 1 was added, 3 g of dry yeast (manufactured by Nisshin Foods Inc., trade name "Nissin Super Kameriya Dry Yeast") and 14 g of shortening (manufactured by J-OIL MILLS, Inc., trade name "FASHIE") were added on the mixed powder so as not to touch the city water. The menu of the automatic home bakery was set to "white bread/baked color: normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (20°C) for 1 hr.

(Sample 2-2)

By the same procedure as in Sample 2-1 except that 0.001U of transglutaminase (manufactured by Ajinomoto Co., Inc., trade name "Activa (registered trade mark)TG") was also added per 1 g of bread flour when a mixed powder was put into the container of a commercially available automatic home bakery, white bread was produced.

(Sample 2-3)

By the same procedure as in Sample 2-2 except that 0.002U of transglutaminase was added per 1 g of bread flour, white bread was produced.

(Sample 2-4)

By the same procedure as in Sample 2-1 except that 1.0U of Thermus thermophilus-derived amylomaltase aqueous solution was added per 1 g of bread flour before a mixed flour was put into a container of a commercially available automatic home bakery, white bread was produced.

(Sample 2-5)

By the same procedure as in Sample 2-4 except that 5.0U of Thermus thermophilus-derived amylomaltase aqueous solution was added per 1 g of bread flour, white bread was produced.

(Sample 2-6)

By the same procedure as in Sample 2-4 except that 0.001U of transglutaminase (manufactured by Ajinomoto Co., Inc., trade name "Activa (registered trade mark)TG") was also added per 1 g of bread flour when a mixed powder was put into the container of a commercially available automatic home bakery, white bread was produced.

(Sample 2-7)

By the same procedure as in Sample 2-6 except that 0.002U of transglutaminase was added per 1 g of bread flour, white bread was produced.

(Sample 2-8)

By the same procedure as in Sample 2-6 except that 5.0U of Thermus thermophilus-derived amylomaltase aqueous solution was added per 1 g of bread flour, white bread was produced.

(Sample 2-9)

By the same procedure as in Sample 2-6 except that 0.002U of transglutaminase was added per 1 g of bread flour and 5.0U of Thermus thermophilus-derived amylomaltase aqueous solution was added per 1 g of bread flour, white bread was produced.

(Measurement of bread height and calculation of bake elongation)

The height of the central portion (also referred to as "bread height" in the present specification) was measured for each of the white breads of Samples 2-1 to 2-9 after allowing to cool at room temperature (20°C) for 1 hr.
From the measured bread height, the bake elongation (%) of the breads of Samples 2-2 to 2-9 was calculated based on the following formula:

```
[Bake elongation (%)] = [Bread height (cm)] ÷ [Bread height
(cm) of Sample 2-1] × 100
```

(Sensory evaluation)

After allowing to cool at room temperature (20°C) for 1 hr, the white breads of Samples 2-1 to 2-9 were sliced to a thickness of 2 cm, tightly sealed in a polyethylene bag, and stored at temperature of 10°C, humidity 50%. After storage for 2 days, an evaluation panel of 4 people ate only the crumb part (internal phase part of white bread) of each white bread, and preference was evaluated based on the same criteria as in Experimental Example 1 and retrogradation feeling, crispness, and meltability in a mouth were evaluated based on the following criteria. The sensory evaluation was performed by a disgorging method.

[Retrogradation feeling]

++++: stronger retrogradation feeling than Sample 2-1
+++: feeling of retrogradation equivalent to Sample 2-1
++: less retrogradation feeling than Sample 2-1
+: clearly less retrogradation feeling than Sample 2-1
±: very low retrogradation feeling compared to Sample 2-1
-: no retrogradation feeling

[Crispness]

++: very good crispness compared to white bread of Sample 2-1
+: good crispness compared to white bread of Sample 2-1
±: crispness equivalent to that of white bread of Sample 2-1
-: poor crispness compared to white bread of Sample 2-1

[Meltability in mouth]

++: very good meltability in mouth compared to white bread of Sample 2-1
+: good meltability in mouth compared to white bread of Sample 2-1
±: meltability in mouth equivalent to that of white bread of Sample 2-1
-: poor meltability in mouth compared to white bread of Sample 2-1

[Preference]

⊙: extremely preferable
O: very preferable
Δ: preferable
×: not preferable

The results are shown in the following Tables 3 and 4. In the Tables, "TG" is transglutaminase and "TtAM" is thermus thermophilus-derived amylomaltase.

[Table 3]

| sample | 2-1 | 2-2 | 2-3 | 2-4 | 2-5 |
|---|---|---|---|---|---|
| TG (unit per 1 g of bread flour) | | 0.001 | 0.002 | | |
| TtAM (unit per 1 g of bread flour) | | | | 1.0 | 5.0 |
| bread height (cm) | 14.0 | 14.2 | 14.1 | 14.2 | 15.1 |
| bake elongation (%) | | 101.4 | 100.7 | 101.4 | 107.9 |
| retrogradation feeling | +++ | +++ | ++++ | ++ | + |
| | texture was hard, strong dry feeling was felt | dry feeling equivalent to Sample 2-1 was felt | stronger dry feeling than Sample 2-1 was felt, texture was hard | dry feeling was suppressed more than Sample 2-1 | clearly soft texture, slight dry feeling |

(continued)

| sample | 2-1 | 2-2 | 2-3 | 2-4 | 2-5 |
|---|---|---|---|---|---|
| crispness | + (standard) | + | ++ | + | - |
| meltability in mouth | ± (standard) | ± | - | ± | - |
| preference | × | × | × | Δ | O |

[Table 4]

| sample | 2-6 | 2-7 | 2-8 | 2-9 |
|---|---|---|---|---|
| TG (unit per 1 g of bread flour) | 0.001 | 0.002 | 0.001 | 0.002 |
| TtAM (unit per 1 g of bread flour) | 1.0 | 1.0 | 5.0 | 5.0 |
| bread height (cm) | 14.4 | 14.3 | 15.3 | 15.2 |
| bake elongation (%) | 102.9 | 102.1 | 109.3 | 108.6 |
| retrogradetion feeling | ± | ± | ± to - | ± |
| | clearly softer texture than Sample 2-4, moist feeling was high | clearly softer texture than Sample 2-4, moist feeling was high | moist feeling was higher than Sample 2-4 | slightly harder texture than Sample 2-8, but moist feeling was high |
| crispness | + | ++ | + | + |
| meltability in mouth | + | ± | + | + |
| preference | ⊙ | ⊙ | ⊙ | ⊙ |

As is clear from the results shown in Tables 3 and 4, a strong retrogradation suppressive effect was confirmed in the white breads of Samples 2-6 to 2-9, and crispness and meltability in a mouth were fine.

On the other hand, a clear retrogradation suppressive effect was not observed in the white bread of Sample 2-2 to which transglutaminase was added alone, and the white bread of Sample 2-3 tended to show hard texture after storage as compared with the white bread of Sample 2-1.

<Experimental Example 3>

The white breads of Samples 2-1, 2-3, 2-7 and 2-9 produced in Example 2 were tightly sealed in a polyethylene bag and stored under the conditions of temperature 10°C, humidity 50% for 2 days, and then a compression test was performed.

The compression test was performed by the following procedures (1) - (3) (n=7 - 9) using a creepmeter (manufactured by YAMADEN co., ltd. "RHEONER II", model number: RE2-33005, probe shape: wedge shape, measurement mode: compressive rupture test).

(1) The crumb of each white bread (internal phase part of white bread) was cut out into a cube with a side of 2 cm and used as a measurement sample.
(2) The measurement sample is placed on the creepmeter such that a probe vertically hits the top surface of the measurement sample.
(3) The measurement sample (Sample temperature: 20°C) is compressed with the probe under the conditions of compression rate 1 mm/sec, compression ratio 99.99%, and the stress (N) at a compression ratio 10% is measured.

The results are shown in Fig. 2.

As is clear from the results shown in Fig. 2, it was confirmed that the white breads of Samples 2-7 and 2-9 maintained softness as compared with the white bread of Sample 2-3 to which transglutaminase was added alone.

<Experimental Example 4>

[Production of white bread]

(Sample 4-1)

After weighing 184.8 g of city water in a container (bread case with blades) of a commercially available automatic home bakery (automatic bread-baking machine for home use) (manufactured by MK Seiko Co., Ltd., product number: HBK-100), a mixed powder (312.2 g) prepared in the same manner as in Experimental Example 1 was added, 3 g of dry yeast (manufactured by Nisshin Foods Inc., trade name "Nissin Super Kameriya Dry Yeast") and 14 g of shortening (manufactured by J-OIL MILLS, Inc., trade name "FASHIE") were added on the mixed powder so as not to touch the city water. The menu of the automatic home bakery was set to "white bread/baked color: normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (20°C) for 1 hr.

(Sample 4-2)

By the same procedure as in Sample 4-1 except that 10U of lipase (manufactured by Yakult Pharmaceutical Industry Co., Ltd., trade name "Lilipase A-10D") was also added per 1 g of bread flour when a mixed powder was put into the container of a commercially available automatic home bakery, white bread was produced.

(Sample 4-3)

By the same procedure as in Sample 4-1 except that 1U of Thermus thermophilus-derived amylomaltase aqueous solution was added per 1 g of bread flour before a mixed flour was put into a container of a commercially available automatic home bakery, white bread was produced.

(Sample 4-4)

By the same procedure as in Sample 4-3 except that 10U of lipase (manufactured by Yakult Pharmaceutical Industry Co., Ltd., trade name "Lilipase A-10D") was also added per 1 g of bread flour when a mixed powder was put into the container of a commercially available automatic home bakery, white bread was produced.

(Sensory evaluation)

After allowing to cool at room temperature (20°C) for 1 hr, the white breads of Samples 4-1 to 4-4 were sliced to a thickness of 2 cm, tightly sealed in a polyethylene bag, and stored at temperature of 10°C, humidity 50%. After storage for 2 days, an evaluation panel of 4 people ate only the crumb part (internal phase part of white bread) of each white bread, and retrogradation feeling, crispness, meltability in a mouth and preference of texture were evaluated based on the following criteria. The sensory evaluation was performed by a disgorging method.

[Retrogradation feeling]

++++: stronger retrogradation feeling than Sample 4-1
+++: feeling of retrogradation equivalent to Sample 4-1
++: less retrogradation feeling than Sample 4-1
+: clearly less retrogradation feeling than Sample 4-1
±: very low retrogradation feeling compared to Sample 4-1
-: no retrogradation feeling

[Crispness]

++: very good crispness compared to white bread of Sample 4-1
+: good crispness compared to white bread of Sample 4-1
±: crispness equivalent to that of white bread of Sample 4-1
-: poor crispness compared to white bread of Sample 4-1

[Meltability in mouth]

++: very good meltability in mouth compared to white bread of Sample 4-1
+: good meltability in mouth compared to white bread of Sample 4-1
±: meltability in mouth equivalent to that of white bread of Sample 4-1
-: poor meltability in mouth compared to white bread of Sample 4-1

[Preference of texture]

⊙: drastically preferable compared to white bread of Sample 4-1

O: preferable compared to white bread of Sample 4-1

Δ: slightly preferable compared to white bread of Sample 4-1

×: same level of preference as white bread of Sample 4-1

As used herein, "preference of texture" refers to the overall preference of texture in consideration of other textures (moist feeling, resilience, smoothness, etc.) in addition to crispness and meltability in mouth.
The results are shown in the following Table 5. In the Table, "TtAM" is thermus thermophilus-derived amylomaltase.

[Table 5]

| sample | 4-1 | 4-2 | 4-3 | 4-4 |
|---|---|---|---|---|
| lipase (unit per 1 g of bread flour) | | 10 | | 10 |
| TtAM (unit per 1 g of bread flour) | | | 1 | 1 |
| retrogradation feeling | +++ | ++ | ++ | ± |
| | texture was hard, strong dry feeling was felt | sofer than Sample 4-1 but strong dry feeling was felt | softer than Sample 4-1, dry feeling was suppressed | clearly soft texture, moist feeling was high |
| crispness | ± (standard) | ± | ± | + |
| meltability in mouth | ± (standard) | ± | + | + |
| preference of texture | × (standard) | Δ | Δ | ⊙ |

(Property measurement)

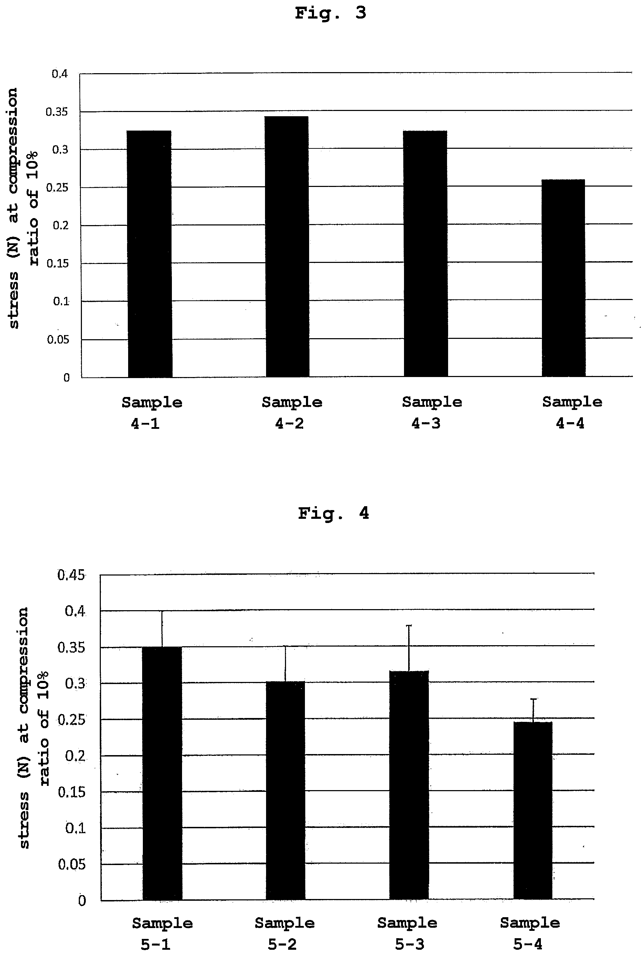

The white breads of Samples 4-1 to 4-4 were tightly sealed in a polyethylene bag and stored under the conditions of temperature 10°C, humidity 50% for 2 days, and then a compression test similar to Experimental Example 3 was performed. The results are shown in Fig. 3.

As is clear from the results shown in Table 5, only a slight retrogradation suppressive effect was found in the white bread of sample 4-2 added with lipase alone, and the crispness, meltability in a mouth and preference of texture were not improved. For example, the moist feeling of the white bread of sample 4-2 was not improved.

The white bread of sample 4-3 added with an amylomaltase derived from Thermus thermophilus alone showed a slight retrogradation suppressive effect, but the crispness, meltability in a mouth and preference of texture were not sufficiently improved. For example, the white bread of sample 4-3 showed an insufficient improvement in the softness and moist feeling.

On the other hand, a strong retrogradation suppressive effect and good crispness and good meltability in a mouth were confirmed in the white bread of sample 4-4. For example, the moist feeling which was insufficient in the white breads of samples 4-2 and 4-3 was clearly improved in the white bread of sample 4-4.

From the results shown in Fig. 3, it was confirmed that softness was maintained in the white bread of sample 4-4 as compared with the white bread of 4-2 added with lipase alone and the white bread of sample 4-3 added with amylomaltase derived from Thermus thermophilus alone.

<Experimental Example 5>

[Production of white bread]

(Sample 5-1)

Bread flour (manufactured by Nippon Flour Mills Co., Ltd., trade name "NIPPN EAGLE (bread flour)"), granulated sugar (manufactured by Mitsui Sugar Co., Ltd., trade name "SPOON brand Granulated sugar"), and cooking salt (manufactured by Naikai Salt Industries Co., LTD., trade name "Nakuru M") were premixed in the amounts shown in the following Table 6 to give a mixed powder.

After weighing 190 g of city water (22.3°C) in a container (bread case with blades) of a commercially available automatic home bakery (automatic bread-baking machine for home use) (manufactured by MK Seiko Co., Ltd., product number: HBK-100), the above-mentioned mixed powder was added, 3 g of dry yeast (manufactured by Nisshin Foods Inc., trade name "Nissin Super Kameriya Dry Yeast") and 20 g of shortening (manufactured by J-OIL MILLS, Inc., trade name "FASHIE") were added on the mixed powder so as not to touch the city water. The menu of the automatic home bakery was set to "white bread/baked color: normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (24.5°C, humidity: 56%) for 1 hr.

[Table 6]

| material | weight (g) |
| --- | --- |
| bread flour | 280 |
| granulated sugar | 15 |
| cooking salt | 4 |

(Sample 5-2)

By the same procedure as in Sample 5-1 except that 1U of Thermus thermophilus-derived amylomaltase aqueous solution was also added per 1 g of bread flour before a mixed flour was put into a container of a commercially available automatic home bakery, white bread was produced.

(Sample 5-3)

By the same procedure as in Sample 5-1 except that 0.4U of protease (manufactured by Amano Enzyme Inc., trade name "Protin SD-NY10") was also added per 1 g of bread flour when a mixed powder was put into the container of a commercially available automatic home bakery, white bread was produced.

(Sample 5-4)

By the same procedure as in Sample 5-2 except that 0.4U of protease (manufactured by Amano Enzyme Inc.,

trade name "Protin SD-NY10") was also added per 1 g of bread flour when a mixed powder was put into the container of a commercially available automatic home bakery, white bread was produced.

(Measurement of bread height and weight)

The height of the central portion (also referred to as "bread height" in the present specification) and weight were measured for each of the white breads of Samples 5-1 to 5-4 after allowing to cool at room temperature (20°C) for 1 hr.

(Sensory evaluation)

After allowing to cool at room temperature (20°C) for 1 hr, the white breads of Samples 5-1 to 5-4 were each sliced to a thickness of 2 cm, tightly sealed in a polyethylene bag, and stored at temperature of 10°C, humidity 50%. After storage for 2 days, an evaluation panel of 4 people ate only the crumb part (internal phase part of white bread) of each white bread, and moist feeling, meltability in a mouth, softness and palatability were each evaluated based on the following criteria. The sensory evaluation was performed by a disgorging method.

[Moist feeling]

++: moist compared to white bread of Sample 5-1
+: slightly moist compared to white bread of Sample 5-1
±: moist feeling equivalent to white bread of Sample 5-1
-: slightly dry compared to white bread of Sample 5-1
--: dry compared to white bread of Sample 5-1

[Meltability in mouth]

++: good meltability in a mouth compared to white bread of Sample 5-1
+: slightly good meltability in a mouth compared to white bread of Sample 5-1
±: meltability in a mouth equivalent to white bread of Sample 5-1
-: slightly sticky compared to white bread of Sample 5-1
--: sticky compared to white bread of Sample 5-1

[Softness]

++: soft compared to white bread of Sample 5-1
+: slightly soft compared to white bread of Sample 5-1
±: softness equivalent to white bread of Sample 5-1
-: slightly hard compared to white bread of Sample 5-1
--: hard compared to white bread of Sample 5-1

[Palatability]

++: preferable compared to white bread of Sample 5-1
+: slightly preferable compared to white bread of Sample 5-1
±: preference (palatability) equivalent to white bread of Sample 5-1
-: slightly unpreferable compared to white bread of Sample 5-1
--: not preferable compared to white bread of Sample 5-1

As used herein, "palatability" refers to overall preference as food.
The results are shown in the following Table 7. In the Table, "TtAM" is thermus thermophilus-derived amylomaltase.

[Table 7]

| sample | 5-1 | 5-2 | 5-3 | 5-4 |
|---|---|---|---|---|
| TtAM (unit per 1 g of bread flour) | | 1 | | 1 |
| Protease (unit per 1 g of bread flour) | | | 0.4 | 0.4 |
| weight (g) | 457.51 | 459.44 | 456.23 | 459.15 |
| bread height (cm) | 14.1 | 13.6 | 16.4 | 15.8 |
| moist feeling | $\pm$ (standard) | + | + | ++ |
| meltability in mouth | $\pm$ (standard) | - | + | + |
| softness | $\pm$ (standard) | - | + | ++ |
| palatability | $\pm$ (standard) | - | + | ++ |

(property measurement)

The white breads of Samples 5-1 to 5-4 were tightly sealed in a polyethylene bag and stored under the conditions of temperature 10°C, humidity 50% for 2 days, and then a compression test similar to Experimental Example 3 was performed. The results are shown in Fig. 4.

As is clear from the results shown in Table 7, the moist feeling, meltability in a mouth, softness and palatability were good in the white bread of sample 5-4.

On the other hand, the meltability in a mouth, softness and palatability decreased in the white bread of sample 5-2 added with an amylomaltase derived from Thermus thermophilus alone. In the white bread of sample 5-3 added with protease alone, a clear retrogradation suppressive effect was not found and the crispness, meltability in a mouth and preference of texture were not improved.

From the results shown in Fig. 4, it was confirmed that the softness was maintained in the white bread of sample 5-4 as compared with the white bread of sample 5-2 added with an amylomaltase derived from Thermus thermophilus alone and the white bread of sample 5-3 added with protease alone.

From these results, it was confirmed that a combined use of an amylomaltase derived from Thermus thermophilus and protease imparts a moist feeling and softness, and bread having preferable texture is obtained.

<Experimental Example 6>

[Production of rice]

(Sample 6-1)

Polished rice (HITOMEBORE from Miyagi Prefecture) (150 g) was weighed, and washed (stirring 20 times $\times$ 5 times). Water was drained and the rice was transferred to a rice cooking pot (inside pot) of a household rice cooker (manufactured by Koizumi Seiki Co., Ltd., Rice Cooker Mini KSC-1511), and water was added such that the total amount of the polished rice and water was 360 g. The rice cooking pot was wrapped, the polished rice was left standing for 1 hr while being immersed in water, and cooked to obtain rice (cooked rice).

(Sample 6-2)

By the same procedure as in Sample 6-1 except that 0.5U of Thermus thermophilus-derived amylomaltase aqueous solution was added per 1 g of polished rice before cooking and after allowing the polished rice to stand while being immersed in water for 1 hr, the rice (cooked rice) was produced.

(Sample 6-3)

By the same procedure as in Sample 6-1 except that 17.25U of transglutaminase (manufactured by Amano Enzyme Inc., activity value per 1 g of product: 1150U) was added per 1 g of polished rice before cooking and after allowing the polished rice to stand while being immersed in water for 1 hr, the rice (cooked rice) was produced. Transglutaminase was added after dissolving transglutaminase in the immersion liquid of the polished rice.

(Sample 6-4)

By the same procedure as in Sample 6-1 except that 0.5U of Thermus thermophilus-derived amylomaltase aqueous solution per 1 g of polished rice and 17.25U of transglutaminase (manufactured by Amano Enzyme Inc., activity value per 1 g of product: 1150U) per 1 g of polished rice were added before cooking and after allowing the polished rice to stand while being immersed in water for 1 hr, the rice (cooked rice) was produced. Transglutaminase was added after dissolving transglutaminase in the immersion liquid of the polished rice.

(Sensory evaluation)

The rice of samples 6-1 to 6-4 was steamed for 15 min after the rice was cooked, the rice cooking pot containing each rice was turned upside down on a vat to transfer the rice to the vat, and each rice was mixed and spread so as not to crush the rice grains and remove the rough heat. The rice that was in contact with the wall of the rice cooking pot was removed. Each mixed and spread rice was lightly wrapped to prevent drying and left standing for about 30 min. After confirming that each rice had cooled, the whole amount was put in a pack, wrapped, and stored in a thermostatic tank set at 15°C.
After storing for 1 day in the thermostatic tank, an evaluation panel of 4 people ate each rice, and the degree of looseness and stickiness in the mouth was evaluated based on the following criteria. The degree of adhered residue to the rice cooking pot after transferring the rice from the rice cooking pot to the vat was confirmed by visual observation by the evaluation panel of 4 people based on the following criteria.

[Looseness and stickiness]

O: moderate easiness in loosening and moderate stickiness
×: no easiness in loosening or stickiness

[Adhered residue]

O: no adhered residue and preferable
×: adhered residue is present and unpreferable

The results are shown in the following Table 8. In the Table, "TtAM" is thermus thermophilus-derived amylomaltase and "TG" is transglutaminase.

[Table 8]

| sample | 6-1 | 6-2 | 6-3 | 6-4 |
|---|---|---|---|---|
| TtAM (unit per 1 g of polished rice) | | 0.5 | | 0.5 |
| TG (unit per 1 g of polished rice) | | | 17.25 | 17.25 |
| evaluation results of adhered residue | O | × | O | O |
| evaluation results of looseness and stickiness | × | × | × | O |

As is clear from the results shown in Table 8, the rice of sample 6-4 had moderate looseness and stickiness, and was free of adhered residue to the pot and preferable.
On the other hand, the rice of sample 6-2 added with an amylomaltase derived from Thermus thermophilus alone had an adhered residue and was preferable. Easiness in loosening was not felt in the rice of sample 6-3 added with transglutaminase alone.
From these results, it was confirmed that a combined use of an amylomaltase derived from Thermus thermophilus and transglutaminase solves the problems in producing rice when the amylomaltase and transglutaminase are each used alone, and affords an appropriate texture modification effect on easiness of loosening and the like of rice.

The effect is particularly useful for foods (e.g., fried rice, pilaf, etc.) requesting prevention of excessive attachment between rice grains.

<Experimental Example 7>

(Sample 7-1)

To commercially available dried potato flakes (manufactured by Kabushiki Kaisha Taimou, trade name "JAGAIMO Flake") (40 g) was added hot water (80°C) to a total amount of 160 g, and the mixture was stirred with a spatula for 3 min to give a uniform mixture. The mixture was divided into 3 packs of 50 g each, vacuum-packed, and heated in a water bath at 75°C for 30 min. After removing the rough heat with running water, the packs were refrigerated at 4°C for 1 day.

(Sample 7-2)

By the same procedure as in sample 7-1 except that 100-fold diluted Thermus thermophilus-derived amylomaltase aqueous solution (146.5 $\mu$L) (unit number of amylomaltase derived from Thermus thermophilus per 1 g of dried potato flakes: 0.1U) was also added when hot water was added to commercially available dried potato flakes to make the total amount 160 g, mashed potato was prepared.

(Sample 7-3)

By the same procedure as in sample 7-1 except that 100-fold diluted Thermus thermophilus-derived amylomaltase aqueous solution (1465.2 $\mu$L) (unit number of amylomaltase derived from Thermus thermophilus per 1 g of dried potato flakes: 1U) was also added when hot water was added to commercially available dried potato flakes to make the total amount 160 g, mashed potato was prepared.

(Sample 7-4)

By the same procedure as in sample 7-1 except that glucose oxidase (manufactured by SHINNIHON CHEMICALS Corporation, trade name "Sumizyme PGO") (0.6 mg) (unit number of glucose oxidase per 1 g of dried potato flakes: 0.03U) was also added when hot water was added to commercially available dried potato flakes to make the total amount 160 g, mashed potato was prepared.

(Sample 7-5)

By the same procedure as in sample 7-1 except that glucose oxidase (manufactured by SHINNIHON CHEMICALS Corporation, trade name "Sumizyme PGO") (6 mg) (unit number of glucose oxidase per 1 g of dried potato flakes: 0.3U) was also added when hot water was added to commercially available dried potato flakes to make the total amount 160 g, mashed potato was prepared.

(Sample 7-6)

By the same procedure as in sample 7-1 except that 100-fold diluted Thermus thermophilus-derived amylomaltase aqueous solution (146.5 $\mu$L) (unit number of amylomaltase derived from Thermus thermophilus per 1 g of dried potato flakes: 0.1U), and glucose oxidase (manufactured by SHINNIHON CHEMICALS Corporation, trade name "Sumizyme PGO") (0.6 mg) (unit number of glucose oxidase per 1 g of dried potato flakes: 0.03U) were also added when hot water was added to commercially available dried potato flakes to make the total amount 160 g, mashed potato was prepared.

(Sample 7-7)

By the same procedure as in sample 7-1 except that 100-fold diluted Thermus thermophilus-derived amylomaltase aqueous solution (146.5 $\mu$L) (unit number of amylomaltase derived from Thermus thermophilus per 1 g of dried potato flakes: 0.1U), and glucose oxidase (manufactured by SHINNIHON CHEMICALS Corporation, trade name "Sumizyme PGO") (6 mg) (unit number of glucose oxidase per 1 g of dried potato flakes: 0.3U) were also added when hot water was added to commercially available dried potato flakes to make the total amount 160 g, mashed potato was prepared.

(Sample 7-8)

By the same procedure as in sample 7-1 except that 100-fold diluted Thermus thermophilus-derived amylomaltase aqueous solution (1465.2 µL) (unit number of amylomaltase derived from Thermus thermophilus per 1 g of dried potato flakes: 1U), and glucose oxidase (manufactured by SHINNIHON CHEMICALS Corporation, trade name "Sumizyme PGO") (0.6 mg) (unit number of glucose oxidase per 1 g of dried potato flakes: 0.03U) were also added when hot water was added to commercially available dried potato flakes to make the total amount 160 g, mashed potato was prepared.

(Sample 7-9)

By the same procedure as in sample 7-1 except that 100-fold diluted Thermus thermophilus-derived amylomaltase aqueous solution (1465.2 µL) (unit number of amylomaltase derived from Thermus thermophilus per 1 g of dried potato flakes: 1U), and glucose oxidase (manufactured by SHINNIHON CHEMICALS Corporation, trade name "Sumizyme PGO") (6 mg) (unit number of glucose oxidase per 1 g of dried potato flakes: 0.3U) were also added when hot water was added to commercially available dried potato flakes to make the total amount 160 g, mashed potato was prepared.

(Sensory evaluation)

After refrigerated storage for 7 days, an evaluation panel of three people ate each mashed potato, and evaluated the flavor (potato feeling) and texture (softness, moist feeling) based on the following criteria.

[Flavor (potato feeling)]

O: preferable potato feeling
Δ: slight potato feeling
×: almost no potato feeling

As used herein, the "potato feeling" refers to aroma and flavor unique to steamed potatoes.

[Texture (softness)]

+++: very soft as compared with mashed potato of sample 7-1
++: soft as compared with mashed potato of sample 7-1
+: slightly soft as compared with mashed potato of sample 7-1
±: softness equivalent to the mashed potato of sample 7-1
-: slightly hard as compared with mashed potato of sample 7-1
--: hard as compared with mashed potato of sample 7-1

[Texture (moist feeling)]

+++: very moist
++: moist as compared with mashed potato of sample 7-1
+: slightly moist as compared with mashed potato of sample 7-1
±: moist feeling equivalent to the mashed potato of sample 7-1
-: slightly dried as compared with mashed potato of sample 7-1
--: dried as compared with mashed potato of sample 7-1

The results are shown in the following Table 9. In the Table, "TtAM" is Thermus thermophilus-derived amylomaltase, and "GO" is glucose oxidase.

[Table 9]

| sample | | 7-1 | 7-2 | 7-3 | 7-4 | 7-5 | 7-6 | 7-7 | 7-8 | 7-9 |
|---|---|---|---|---|---|---|---|---|---|---|
| TtAM (unit per 1 g of dried potato flakes) | | | 0.1 | 1 | | | 0.1 | 0.1 | 1 | 1 |
| GO (unit per 1 g of dried potato flakes) | | | | | 0.03 | 0.3 | 0.03 | 0.3 | 0.03 | 0.3 |
| flavor (potato feeling) | | × | × | × | × | × | O | Δ | Δ | O |
| texture | softness | ± (standard) | ++ | +++ | - | - | ++ | ++ | +++ | +++ |
| | moist feeling | ± (standard) | ++ | +++ | + | ± | ++ | ++ | +++ | +++ |

As is clear from the results shown in Table 9, it was confirmed that mashed potato can maintain good texture (softness, moist feeling) and flavor (potato feeling) even after refrigerated storage for 7 days by a combined use of a Thermus thermophilus-derived amylomaltase and glucose oxidase (Samples 7-6 to 7-9).

<Experimental Example 8> Retrogradation evaluation of starch (Turbidity method)

In this test, the retrogradation evaluation of starch was performed according to and by modifying the method described in JP-A-2001-333712. The starch immediately after gelatinization is transparent, and a gelatinized starch liquid obtained by dissolving the starch immediately after gelatinization in water is transparent. On the other hand, retrograded starch becomes a white insoluble material, and the gelatinized starch liquid becomes cloudy with time. The degree of cloudiness (turbidity) of the gelatinized starch liquid was measured at a wavelength of 530 nm using a spectrophotometer, and the amount of change over time was determined to evaluate retrogradation of the starch.

In the method described in JP-A-2001-333712, a starch paste of corn starch was prepared, and changes in the turbidity were determined by measuring the absorbance at a wavelength of 720 nm. In the rice starch used in this test, the absorbance at a wavelength of 530 nm is higher than that at a wavelength of 720 nm. Therefore, the measurement wavelength was modified in this test so that changes in the turbidity could be easily captured.

[Preparation of 2% gelatinized starch liquid]

Ultrapure water (Milli-Q water) (98 mL) was measured, dispensed into a 200 mL beaker, and 2 g of rice starch was added while stirring the mixture with a stirrer. The obtained rice starch liquid was sufficiently stirred, transferred to a standing pouch, and the pouch was sealed by evacuating the air in the pouch as much as possible and then bathed in a hot water bath at 100°C for 30 min. Then, the pouch was returned to room temperature to obtain a 2% gelatinized starch liquid.

[Preparation of enzyme-treated starch liquids of Experimental plots 2-1 to 6-2, Comparison plot 1-2, and enzyme-untreated starch liquids of Comparison plots 1-1 to 6-2]

A 2% gelatinized starch liquid was mixed with ultrapure water (Milli-Q water) such that a rice starch concentration after mixing was the concentration (1%) described in the following Table 11, or each sugar solution (glucose liquid, maltose liquid, maltotriose liquid, DE8 dextrin liquid, DE4 dextrin liquid) described in the following Table 11 was mixed such that a rice starch concentration and a sugar concentration after mixing were the concentration (1%) described in the following Table 11. TtAM was added to each of the obtained mixtures at 1U per 1 g of the rice starch, sufficiently stirred in a vortex mixer and heated for 1 hr in a water bath at 70°C. Then, the enzyme (TtAM) was deactivated by heating for 10 min at 100°C by an IH heater, and then returned to room temperature to obtain the enzyme-treated starch liquids of Experimental plots 2-1 to 6-2 and Comparison plot 1-2.

By the same procedure as in the above except that TtAM was not added to the mixture, the enzyme-untreated starch liquids of Comparison plots 1-1 to 6-2 were obtained.

In this test, as the dextrin with DE of 8, "Pinedex #1" manufactured by Matsutani Chemical Industry Co., Ltd. was used. As the dextrin with DE of 4, "Pinedex #100" manufactured by Matsutani Chemical Industry Co., Ltd. was used.

[Measurement of absorbance]

Respective enzyme-treated starch liquid and enzyme-untreated starch liquid were dispensed into 96-well plates

by 300 μL (n=2) within 1 hr from preparation and the absorbance at a wavelength of 530 nm was measured using a microplate reader (manufactured by Molecular Devices Japan). In the following, the thus-measured absorbance is also referred to as "day0 absorbance".

A plate seal was attached to a 96-well plate after day0 absorbance measurement, the plate was refrigerated for 24 hr at 5°C, returned to room temperature and stirred, and the absorbance at a wavelength of 530 nm was measured again using a microplate reader (manufactured by Molecular Devices Japan). The thus-measured absorbance is also referred to as "day1 absorbance" in the following.

[Calculation of retrogradation degree]

For evaluation of retrogradation of each enzyme-treated starch liquid and enzyme-untreated starch liquid, the difference between the day1 absorbance and the day0 absorbance was calculated as an index of retrogradation over time as shown in the following formula, and evaluation was performed based on the criteria shown in Table 10.

index of starch retrogradation with time lapse = day1 absorbance of each Experimental plot or Comparison plot - day0 absorbance of each Experimental plot or Comparison plot

[Table 10]

| index of starch retrogradation with time lapse | evaluation |
|---|---|
| less than 0.040 | ⊙ (retrogradation was remarkably suppressed) |
| not less than 0.040 and less than 0.050 | O (retrogradation was clearly suppressed) |
| not less than 0.050 and less than 0.090 | Δ retrogradation was suppressed) |
| not less than 0.090 and less than 0.094 | - (retrogradation was not suppressed) |
| not less than 0.094 | x (retrogradation was promoted) |

The results are shown in the following Table 11. In the Table, "U/g-st" means "unit number per 1 g of starch".

Table 11]

| | | | | absorbance (turbidity) at wavelength 530 nm | | index of retrogradation with time lapse | evaluation |
|---|---|---|---|---|---|---|---|
| rice starch | TtAM (U/g-st) | - | Experimental plot | day0 | day1 | day1-day0 | |
| 1% | - | - | Comparison plot 1-1 | 0.409 | 0.503 | 0.094 | - |
| | 1 | - | Comparison plot 1-2 | 0.401 | 0.442 | 0.041 | O |
| | TtAM (U/g-st) | glucose (%) | Experimental plot | day0 | day1 | day1-day0 | |
| | - | 0.005 | Comparison plot 2-1 | 0.367 | 0.438 | 0.071 | Δ |
| | | 0.05 | Comparison plot 2-2 | 0.368 | 0.449 | 0.081 | Δ |
| | | 0.5 | Comparison plot 2-3 | 0.356 | 0.423 | 0.067 | Δ |
| | 1 | 0.005 | Experimental plot 2-1 | 0.360 | 0.384 | 0.023 | ⊙ |
| | | 0.05 | Experimental plot 2-2 | 0.315 | 0.342 | 0.027 | ⊙ |
| | | 0.5 | Experimental plot 2-3 | 0.227 | 0.237 | 0.010 | ⊙ |
| | TtAM (U/g-st) | maltose (%) | Experimental plot | day0 | day1 | day1-day0 | |
| | - | 0.005 | Comparison plot 3-1 | 0.414 | 0.515 | 0.101 | × |
| | | 0.05 | Comparison plot 3-2 | 0.426 | 0.515 | 0.089 | Δ |
| | | 0.5 | Comparison plot 3-3 | 0.406 | 0.490 | 0.084 | Δ |

(continued)

| rice starch | TtAM (U/g-st) | - | Experimental plot | day0 | day1 | day1-day0 | |
|---|---|---|---|---|---|---|---|
| | 1 | 0.005 | Experimental plot 3-1 | 0.411 | 0.435 | 0.024 | ⊙ |
| | | 0.05 | Experimental plot 3-2 | 0.405 | 0.423 | 0.018 | ⊙ |
| | | 0.5 | Experimental plot 3-3 | 0.399 | 0.414 | 0.015 | ⊙ |
| | TtAM (U/g-st) | maltotriose (%) | Experimental plot | day0 | day1 | day1-day0 | |
| | - | 0.005 | Comparison plot 4-1 | 0.361 | 0.429 | 0.068 | Δ |
| | | 0.05 | Comparison plot 4-2 | 0.365 | 0.434 | 0.069 | Δ |
| | | 0.5 | Comparison plot 4-3 | 0.372 | 0.438 | 0.066 | Δ |
| | 1 | 0.005 | Experimental plot 4-1 | 0.351 | 0.366 | 0.015 | ⊙ |
| | | 0.05 | Experimental plot 4-2 | 0.335 | 0.357 | 0.022 | ⊙ |
| | | 0.5 | Experimental plot 4-3 | 0.305 | 0.330 | 0.025 | ⊙ |
| | TtAM (U/g-st) | dextrin (DE8) (%) | Experimental plot | day0 | day1 | day1-day0 | |
| | - | 0.005 | Comparison plot 5-1 | 0.362 | 0.433 | 0.071 | Δ |
| | | 0.05 | Comparison plot 5-2 | 0.373 | 0.444 | 0.071 | Δ |
| | 1 | 0.005 | Experimental plot 5-1 | 0.364 | 0.384 | 0.020 | ⊙ |
| | | 0.05 | Experimental plot 5-2 | 0.349 | 0.376 | 0.027 | ⊙ |
| | TtAM (U/g-st) | dextrin (DE4) (%) | Experimental plot | day0 | day1 | day1-day0 | |
| | - | 0.005 | Comparison plot 6-1 | 0.378 | 0.451 | 0.072 | Δ |
| | | 0.05 | Comparison plot 6-2 | 0.373 | 0.443 | 0.071 | Δ |
| | 1 | 0.005 | Experimental plot 6-1 | 0.385 | 0.405 | 0.020 | ⊙ |
| | | 0.05 | Experimental plot 6-2 | 0.370 | 0.390 | 0.020 | ⊙ |

Column headers: absorbance (turbidity) at wavelength 530 nm (day0, day1); index of retrogradation with time lapse (day1-day0); evaluation

As is clear from the results shown in Table 11, it was confirmed that turbidity increase (day1 absorbance - day0 absorbance) was suppressed, and retrogradation of starch was suppressed by TtAM in Comparison plot 1-2 as compared with Comparison plot 1-1.

In all of Comparison plot 2-1, Comparison plot 2-2 and Comparison plot 2-3 in which glucose was added, an increase in the turbidity was suppressed as compared with Comparison plot 1-1, and suppression of retrogradation by glucose was confirmed.

In all of Experimental plot 2-1, Experimental plot 2-2 and Experimental plot 2-3 in which TtAM and glucose were added, an increase in the turbidity was suppressed as compared with Comparison plot 1-1. In Experimental plot 2-1, Experimental plot 2-2 and Experimental plot 2-3, an increase in the turbidity was small as compared with Comparison plot 1-2 in which the same amount of TtAM was added. Therefore, it was confirmed that the retrogradation suppressive effect of TtAM is synergistically enhanced by a combined use of glucose.

In Comparison plot 3-2 and Comparison plot 3-3 in which maltose was added, an increase in the turbidity was suppressed as compared with Comparison plot 1-1. However, in Comparison plot 3-1, a remarkable retrogradation suppressive effect by starch was not confirmed.

In all of Experimental plot 3-1, Experimental plot 3-2 and Experimental plot 3-3 in which TtAM and maltose were added, an increase in the turbidity was suppressed as compared with Comparison plot 1-1. In Experimental plot 3-1, Experimental plot 3-2 and Experimental plot 3-3, an increase in the turbidity was suppressed as compared with Comparison plot 1-2 in which the same amount of TtAM was added. Therefore, it was confirmed that the retrogradation suppressive effect of TtAM is synergistically enhanced by a combined use of maltose.

In all of Comparison plot 4-1, Comparison plot 4-2 and Comparison plot 4-3 in which maltotriose was added, an increase in the turbidity was suppressed as compared with Comparison plot 1-1, and suppression of retrogradation by maltotriose was confirmed.

In all of Experimental plot 4-1, Experimental plot 4-2 and Experimental plot 4-3 in which TtAM and maltotriose were added, an increase in the turbidity was suppressed as compared with Comparison plot 1-1. In Experimental plot 4-1, Experimental plot 4-2 and Experimental plot 4-3, an increase in the turbidity was small as compared with Comparison plot 1-2 in which the same amount of TtAM was added. Therefore, it was confirmed that the retrogradation suppressive effect of TtAM is synergistically enhanced by a combined use of maltotriose.

In all of Comparison plot 5-1, Comparison plot 5-2, Comparison plot 6-1 and Comparison plot 6-2 in which dextrin (DE8 or DE4)was added, an increase in the turbidity was suppressed as compared with Comparison plot 1-1, and suppression of retrogradation by dextrin was confirmed.

In all of Experimental plot 5-1, Experimental plot 5-2, Experimental plot 6-1 and Experimental plot 6-2 in which TtAM and dextrin (DE8 or DE4) were added, an increase in the turbidity was suppressed as compared with Comparison plot 1-1. In all of Experimental plot 5-1, Experimental plot 5-2, Experimental plot 6-1 and Experimental plot 6-2, an increase in the turbidity was small as compared with Comparison plot 1-2 in which the same amount of TtAM was added. Therefore, it was confirmed that the retrogradation suppressive effect of TtAM is synergistically enhanced by a combined use of dextrin.

<Experimental Example 9>

[Comparison plot 7-1]

Three-fold weight of hot water (80°C) was added to commercially available potato flakes (manufactured by Kabushiki Kaisha Taimou, trade name "JAGAIMO Flake"). After stirring with a spoon for 5 min, the mixture was divided into small portions, vacuum-packed, and heated in a water bath at 75°C for 30 min.

[Comparison plot 7-2]

Three-fold weight of hot water (80°C) was added to commercially available potato flakes (manufactured by Kabushiki Kaisha Taimou, trade name "JAGAIMO Flake"), and then TtAM in an amount of 0.1U or 0.05U per 1 g of dried potato flakes was added. After stirring with a spoon for 5 min, the mixture was divided into small portions, vacuum-packed, and heated in a water bath at 75°C for 30 min.

[Comparison plots 7-3 to 7-5]

Three-fold weight of hot water (80°C) and a starch degradation product (glucose, maltose, dextrin with DE of 8 (manufactured by Matsutani Chemical Industry Co., Ltd., trade name "Pinedex #1")) at 5 wt% of dried potato flakes were added to commercially available potato flakes (manufactured by Kabushiki Kaisha Taimou, trade name "JAGAIMO Flake"). After stirring with a spoon for 5 min, the mixture was divided into small portions, vacuum-packed, and heated in a water bath at 75°C for 30 min.

[Experimental plots 7-1 to 7-6]

Three-fold weight of hot water (80°C) and a starch degradation product (glucose, maltose, dextrin with DE of 8 (manufactured by Matsutani Chemical Industry Co., Ltd., trade name "Pinedex #1")) at 1.5 wt% or 5 wt% of dried potato flakes were added to commercially available potato flakes (manufactured by Kabushiki Kaisha Taimou, trade name "JAGAIMO Flake"), and then TtAM in an amount of 0.05U per 1 g of dried potato flakes was added. After stirring with a spoon for 5 min, the mixture was divided into small portions, vacuum-packed, and heated in a water bath at 75°C for 30 min.

Each mashed potato obtained in the Experimental plots 7-1 to 7-6 and Comparison plots 7-1 to 7-5 was stored at 5°C after heating, and sensory evaluation and property (hardness) measurement were performed by the following methods 1 day, 8 days and 15 days after the start of storage.

(Sensory evaluation)

Each mashed potato of the Experimental plots 7-1 to 7-6 and Comparison plots 7-1 to 7-5 was returned to room temperature. An evaluation panel of 4 people ate them, compared "retrogradation suppressive effect" and "moist feeling" of each mashed potato of the Experimental plots 7-1 to 7-6 and Comparison plots 7-1 to 7-5 with those of the mashed potato of Comparison plot 7-1, and evaluated based on the following criteria. The "retrogradation suppressive effect" in this test refers to an effect of maintaining smoothness by suppressing the texture from becoming dry and crumbly over time or becoming rough.

In addition, as "comprehensive evaluation", evaluation was performed based on the following criteria in comprehensive consideration of the evaluation results of the "retrogradation suppressive effect" and "moist feeling".

[Retrogradation suppressive effect]

    +++: retrogradation is clearly suppressed
    ++: retrogradation is suppressed
    +: slight retrogradation suppressive effect is felt
    -: clear retrogradation suppressive effect is not found
    --: not preferable compared with Comparison plot 7-1

[Moist feeling]

    +++: considerably moist
    ++: moist
    +: slightly moist
    -: clear moist feeling is not found
    --: not preferable compared with Comparison plot 7-1

[Comprehensive evaluation]

    +++: very preferable
    ++: preferable
    +: slightly preferable
    -: slightly unpreferable
    --: not preferable

The results after 8 days after the start of storage are shown in the following Table 12.

[Table 12]

| | amount of TtAM added (unit number per 1 g of potato flakes) | kind and amount added (ratio to potato flakes (weight %)) of starchdegradation product | | comprehensive evaluation | retro-gradation suppressive effect | moist feeling | evaluation comment |
|---|---|---|---|---|---|---|---|
| Comparison plot 7-1 | | | | standard | standard | standard | dry and crumbly, and retrograded |
| Comparison plot 7-2 | 0.05 | | | + | + | + | soft and smooth, but slightly dry and crumbly |
| Comparison plot 7-3 | | glucose | 5 | -- | -- | - | hard and in gel state |
| Experimental plot 7-1 | 0.05 | glucose | 1.5 | + | + | ++ | the same level of softness as Comparison plot 7-2 but moist |
| Experimental plot 7-2 | 0.05 | glucose | 5 | ++ | + | ++ | the same level of softness as Comparison plot 7-2 but moist |
| Comparison plot 7-4 | | Maltose | 5 | -- | - | - | hard and in gel state, with resilience |
| Experimental plot 7-3 | 0.05 | Maltose | 1.5 | ++ | ++ | ++ | softer than Comparison plot 7-2 and moist |
| Experimental plot 7-4 | 0.05 | maltose | 5 | +++ | +++ | +++ | softer than Comparison plot 7-2 and moist; smoother than in Experimental plot 7-3 |
| Comparison plot 7-5 | | dextrin | 5 | -- | -- | - | hard and in gel state |
| Experimental plot 7-5 | 0.05 | Dextrin | 1.5 | ++ | ++ | + | softer than in Comparison plot 7-2 |

(continued)

| | amount of TtAM added (unit number per 1 g of potato flakes) | kind and amount added (ratio to potato flakes (weight %)) of starch degradation product | | comprehensive evaluation | retro-gradation suppressive effect | moist feeling | evaluation comment |
|---|---|---|---|---|---|---|---|
| Experimental plot 7-6 | 0.05 | dextrin | 5 | ++ | ++ | + | softer than in Comparison plot 7-2, and had soft and flaky feeling |

(property measurement)

The hardness of each of the mashed potatoes of Experimental plots 7-1 to 7-6 and Comparison plots 7-1 to 7-5 was measured using a texture analyzer (manufactured by Stable Micro Systems, model number: TA-XT Plus) and by the following procedures (1) - (2).

(1) the prepared mashed potato is dispensed to the edge of a 24-well flat-bottomed microplate (12 mm in diameter, 15 mm in height), covered with a lid, and stored at 5°C.
(2) Using a stainless steel spherical plunger with a diameter of 5 mm, the center of the mashed potato filled in a 24-well flat-bottomed microplate is compressed (pierced) by 50% at a compression rate of 0.5 mm/sec, and the stress 1 second after compression is recorded.

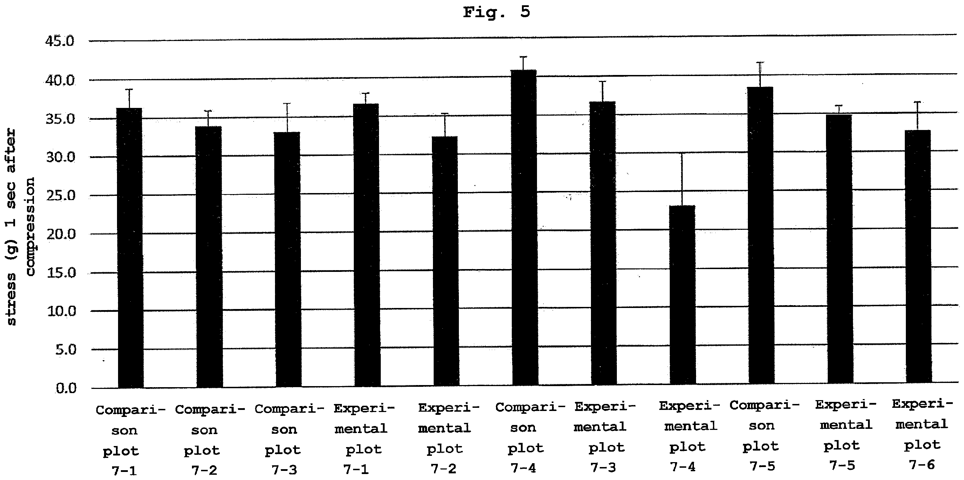

The results 8 days after the start of storage are shown in Fig. 5.
As is clear from the results shown in Table 12, a retrogradation suppressive effect was confirmed in Comparison plot 7-2 in which TtAM was added at 0.05U per 1 g of dried potato flakes.
In all of Experimental plot 7-1 in which TtAM was added at 0.05U per 1 g of dried potato flakes and glucose was added at 1.5 wt% of dried potato flakes, Experimental plot 7-2 in which TtAM was added at 0.05U per 1 g of dried potato flakes and glucose was added at 5 wt% of dried potato flakes, Experimental plot 7-3 in which TtAM was added at 0.05U per 1 g of dried potato flakes and maltose was added at 1.5 wt% of dried potato flakes, Experimental plot 7-4 in which TtAM was added at 0.05U per 1 g of dried potato flakes and maltose was added at 5 wt% of dried potato flakes, Experimental plot 7-5 in which TtAM was added at 0.05U per 1 g of dried potato flakes and dextrin with DE of 8 was added at 1.5 wt% of dried potato flakes, and Experimental plot 7-6 in which TtAM was added at 0.05U per 1 g of dried potato flakes and dextrin with DE of 8 was added at 5 wt% of dried potato flakes, a clearer retrogradation suppressive effect was confirmed as compared with Comparison plot 7-2.
On the other hand, in Comparison plot 7-3 in which glucose was added at 5 wt% of dried potato flakes, Comparison plot 7-4 in which maltose was added at 5 wt% of dried potato flakes, and Comparison plot 7-5 in which dextrin with DE of 8 was added at 5 wt% of dried potato flakes, a clear retrogradation suppressive effect was not confirmed.
In addition, as is clear from the results shown in Fig. 5, it was confirmed that hardness decreased in Experimental plot 7-2 in which TtAM was added at 0.05U per 1 g of dried potato flakes compared with Comparison plot 7-1. Also, it was confirmed that hardness decreased in Experimental plot 7-4 in which TtAM was added at 0.05U per 1 g of dried potato flakes and maltose was added at 5 wt% of dried potato flakes compared with Comparison plot 7-2 in which TtAM was added at 0.05U per 1 g of dried potato flakes.

<Experimental Example 10>

[Production of white bread]

(Comparison plot 8-1)

Bread flour (manufactured by Nippon Flour Mills Co., Ltd., trade name "NIPPN EAGLE (bread flour)") (280 g), granulated sugar (manufactured by Mitsui Sugar Co., Ltd., trade name "SPOON brand Granulated sugar") (15 g), and cooking salt (manufactured by Naikai Salt Industries Co., LTD., trade name "Nakuru M") (4 g) were premixed to give a mixed powder.

After weighing 190 g of city water in a container (bread case with blades) of a commercially available automatic home bakery (automatic bread-baking machine for home use) (manufactured by MK Seiko Co., Ltd., product number: HBK-100), the above-mentioned mixed powder was added, 3 g of dry yeast (manufactured by Nisshin Foods Inc., trade name "Nissin Super Kameriya Dry Yeast") and 20 g of shortening (manufactured by J-OIL MILLS, Inc., trade name "FASHIE") were added on the mixed powder so as not to touch the city water. The menu of the automatic home bakery was set to "white bread" (menu NO: 1) and the baked color was set to "normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (23 - 28°C) for 1 hr.

(Comparison plot 8-2)

By the same procedure as in Comparison plot 8-1 except that 5 wt% of glucose was also added to bread flour when a mixed powder was put into the container of a commercially available automatic home bakery, white bread was produced.

(Comparison plot 8-3)

By the same procedure as in Comparison plot 8-1 except that 5 wt% of maltose was also added to bread flour when a mixed powder was put into the container of a commercially available automatic home bakery, white bread was produced.

(Comparison plot 8-4)

By the same procedure as in Comparison plot 8-1 except that TtAM in an amount of 1U per 1 g of bread flour was also added when a mixed powder was put into the container of a commercially available automatic home bakery, white bread was produced.

(Experimental plot 8-1)

By the same procedure as in Comparison plot 8-1 except that TtAM in an amount of 1U per 1 g of bread flour and 5 wt% of glucose with respect to the bread flour were also added when a mixed powder was put into the container of a commercially available automatic home bakery, white bread was produced.

(Experimental plot 8-2)

By the same procedure as in Comparison plot 8-1 except that TtAM in an amount of 1U per 1 g of bread flour and 5 wt% of maltose with respect to the bread flour were also added when a mixed powder was put into the container of a commercially available automatic home bakery, white bread was produced.

(Property measurement)

The hardness of the white breads of Experimental plot 8-1, Experimental plot 8-2 and Comparison plots 8-1 to 8-4 was measured using a creepmeter (manufactured by YAMADEN co., ltd., "RHEONER II", model number: RE2-33005) and by the following procedures (1) - (4).

(1) White bread is tightly sealed in a polyethylene bag and stored under the conditions of temperature 10°C, humidity 50% for 2 days.
(2) The white bread after storage for 2 days was sliced to a thickness of about 2 cm, 9 points in the center are cut out into a cube with a side of 2 cm, and used as measurement samples.
(3) The measurement sample is placed on the creepmeter such that a plastic wedge-shaped jig (No.49) vertically hits the top surface of the measurement sample.
(4) The measurement sample (Sample temperature: 20°C) is compressed with plastic wedge-shaped jig under the conditions of load cell: 20N, storage pitch: 0.06 sec, compression rate 1 mm/sec, compression ratio 99.99%, and the load value (N) at a compression ratio 50% is measured as an index of the hardness of the white bread. The lower the load value at 50% compression, the softer the white bread tends to be.

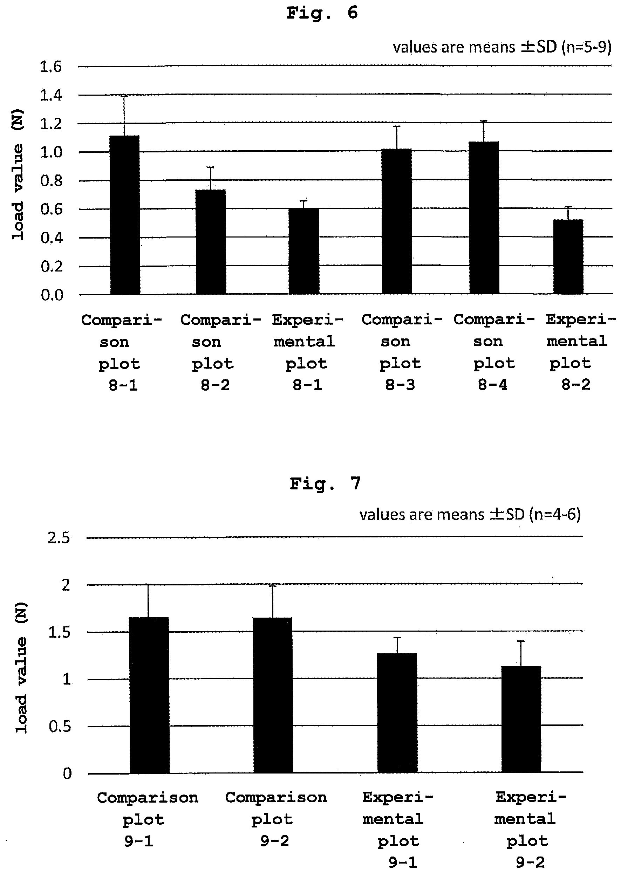

The results are shown in Fig. 6.

(Sensory evaluation)

After allowing to cool at room temperature (23 - 28°C) for 1 hr, the white breads of Experimental plot 8-1, Experimental plot 8-2 and Comparison plots 8-1 to 8-4 were each sliced to a thickness of 2 cm, tightly sealed in a polyethylene bag, and stored at temperature of 10°C, humidity 50% for 2 days. After storage for 2 days, an evaluation panel of 4 people ate only the crumb part (internal phase part of white bread) of each white bread, and a retrogradation suppressive effect on the white breads of Experimental plot 8-1, Experimental plot 8-2 and Comparison plots 8-2 to 8-4 was evaluated based on the following criteria. The sensory evaluation was performed by a disgorging method. The "retrogradation suppressive effect" in this test refers to an effect of maintaining softness and moist feeling by suppressing the texture from becoming dry, or dry and crumbly over time.

[Evaluation criteria]

◎: remarkable retrogradation suppressive effect
O: retrogradation suppressive effect
Δ: weak retrogradation suppressive effect
-: no retrogradation suppressive effect

The results are shown in the following Table 13.

[Table 13]

| | amount of TtAM added (unit number per 1 g of potato flakes) | kind and amount added (ratio to potato flakes (weight %)) of starch degradation product | | retrogradation suppressive effect | evaluation comment |
|---|---|---|---|---|---|
| Comparison plot 8-1 | | | | - | dry feeling: hardness was felt from the start of biting |
| Comparison plot 8-2 | | glucose | 5 | O | heavy (impression of retaining water), moist |
| Comparison plot 8-3 | | maltose | 5 | O | heavy (impression of retaining water), moist |
| Comparison plot 8-4 | 1 | | | O | retrogradation was suppressed compared with Comparison plot 8-1 |
| Experimental plot 8-1 | 1 | glucose | 5 | O - ◎ | moist but light, and retrogradation was suppressed |
| Experimental plot 8-2 | 1 | maltose | 5 | ◎ | soft and moist; light and retrogradation was suppressed more |

As is clear from the results shown in Table 13, a retrogradation suppressive effect was confirmed in Comparison plot 8-4 in which TtAM was added at 1U per 1 g of bread flour.
In addition, a clearer retrogradation suppressive effect was confirmed in Experimental plot 8-1 in which TtAM was added at 1U per 1 g of bread flour and glucose was added at 5 wt% of bread flour, and Experimental plot 8-2 in which TtAM was added at 1U per 1 g of bread flour and maltose was added at 5 wt% of bread flour as compared with Comparison plot 8-4.

<Experimental Example 11>

[Production of white bread]

(Comparison plot 9-1)

Bread flour (manufactured by Nippon Flour Mills Co., Ltd., trade name "NIPPN EAGLE (bread flour)") (280 g), granulated sugar (manufactured by Mitsui Sugar Co., Ltd., trade name "SPOON brand Granulated sugar") (15 g), and cooking salt (manufactured by Naikai Salt Industries Co., LTD., trade name "Nakuru M") (4 g) were premixed to give a mixed powder.

After weighing 190 g of city water in a container (bread case with blades) of a commercially available automatic home bakery (automatic bread-baking machine for home use) (manufactured by MK Seiko Co., Ltd., product number: HBK-100), the above-mentioned mixed powder was added, 3 g of dry yeast (manufactured by Nisshin Foods Inc., trade name "Nissin Super Kameriya Dry Yeast") and 20 g of shortening (manufactured by J-OIL MILLS, Inc., trade name "FASHIE") were added on the mixed powder so as not to touch the city water. The menu of the automatic home bakery was set to "white bread" (menu NO: 1) and the baked color was set to "normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (23 - 28°C) for 1 hr.

(Comparison plot 9-2)

By the same procedure as in Comparison plot 9-1 except that TtAM in an amount of 1U per 1 g of bread flour was also added when a mixed powder was put into the container of a commercially available automatic home bakery, white bread was produced.

(Experimental plot 9-1)

By the same procedure as in Comparison plot 9-1 except that TtAM in an amount of 1U per 1 g of bread flour, 5 wt% of maltose with respect to the bread flour, and 0.012 wt% of glucose with respect to the bread flour were also added when a mixed powder was put into the container of a commercially available automatic home bakery, white bread was produced.

(Experimental plot 9-2)

By the same procedure as in Comparison plot 9-1 except that TtAM in an amount of 1U per 1 g of bread flour, 5 wt% of maltose with respect to the bread flour, 0.012 wt% of glucose with respect to the bread flour, and glucose oxidase in an amount of 0.024U per 1 g of the bread flour were also added when a mixed powder was put into the container of a commercially available automatic home bakery, white bread was produced.

(Property measurement)

The hardness of the white breads of Experimental plot 9-1, Experimental plot 9-2 and Comparison plot 9-1 was measured using a creepmeter (manufactured by YAMADEN co., ltd., "RHEONER II", model number: RE2-33005) and by the same procedures shown in Experimental Example 4.

The results are shown in Fig. 7.

(Sensory evaluation)

After allowing to cool at room temperature (23 - 28°C) for 1 hr, the white breads of Experimental plot 9-1, Experimental plot 9-2 and Comparison plot 9-1 were each sliced to a thickness of 2 cm, tightly sealed in a polyethylene bag, and stored at temperature of 10°C, humidity 50% for 2 days. After storage for 2 days, an evaluation panel A - D of 4 people ate only the crumb part (internal phase part of white bread) of each white bread, and "retrogradation suppressive effect", "crispness", "meltability in a mouth", and "preference" were each scored in 0.5 point increments based on the following criteria. The sensory evaluation was performed by a disgorging method.

2 points: very good
1 point: good

0 point: normal (standard)
-1 point: bad
-2 point: very bad

The results (average of scores of the evaluation panel of 4 people, comments) are shown in the following Table 14. In the Table, "GO" means glucose oxidase.

The scores of each evaluation panel are shown in the following Table 15.

[Table 14]

| | | retrogradation suppressive effect | crisp-ness | meltability in mouth | preference | comment |
|---|---|---|---|---|---|---|
| Comparison plot 9-1 | | 0 | 0 | 0 | 0 | dry and crumbly since retrogradation had proceeded |
| Comparison plot 9-2 | TtAM 1U | 0.38 | 0.5 | -0.63 | 0.25 | retrogradation was suppressed compared with Comparison plot 9-1, but hard |
| Experimental plot 9-1 | TtAM 1U maltose 5% % | 2.0 | 0.5 | 1.0 | 2.0 | moist and soft |
| Experimental plot 9-2 | TtAM 1U maltose 5% GO 0.024U | 2.0 | 1.0 | 1.0 | 2.0 | soft and moist, and preferable |

[Table 15]

| | evaluation panel | retro-gradation suppres-sive effect | crispness | meltability in mouth | preference |
|---|---|---|---|---|---|
| Comparison plot 9-1 | A | 0 | 0 | 0 | 0 |
| | B | 0 | 0 | 0 | 0 |
| | C | 0 | 0 | 0 | 0 |
| | D | 0 | 0 | 0 | 0 |
| Comparison plot 9-2 | A | 1 | 1 | 1 | 1 |
| | B | -1 | 1 | -2 | -1 |
| | C | 0.5 | 0 | -1 | 1 |
| | D | 1 | 0 | -0.5 | 0 |
| Experimental plot 9-1 | A | 2 | 1 | 1 | 2 |
| | B | 2 | 1 | 2 | 2 |
| | C | 2 | 0 | 0 | 2 |
| | D | 2 | 0 | 1 | 2 |
| Experimental plot 9-2 | A | 2 | 1 | 1 | 2 |
| | B | 2 | 2 | 2 | 2 |
| | C | 2 | 1 | 0 | 2 |
| | D | 2 | 0 | 1 | 2 |

As is clear from the results shown in Table 14, a retrogradation suppressive effect was confirmed in Comparison plot 9-2 in which TtAM was added at 1U per 1 g of bread flour.

In addition, in both Experimental plot 9-1 in which TtAM was added at 1U per 1 g of bread flour and maltose was added at 5 wt% of bread flour, and Experimental plot 9-2 in which TtAM was added at 1U per 1 g of bread flour, maltose was added at 5 wt% of bread flour, and glucose oxidase was added, a clearer retrogradation suppressive effect was confirmed as compared with Comparison plot 9-2. The white bread of Experimental plot 9-2 had improved crispness and was more preferable than the white bread of Experimental plot 9-1.

<Experimental Example 12>

[Preparation of 5% starch liquid (Comparison plot 10-1)]

As shown in Table 16, potato starch (manufactured by Nacalai Tesque, Inc.) was measured in an aluminum cup for a rapid visco analyzer (hereinafter to be also simply referred to as "RVA"), and water (Milli-Q water) was added to obtain 5% starch liquid (25 g).

[Preparation of enzyme-added starch liquids of Comparison plot 10-2 and Comparison plot 10-3]

As shown in Table 16, potato starch (manufactured by Nacalai Tesque, Inc.) was measured in an aluminum cup for RVA, water (Milli-Q water) was added, and diluted Thermus thermophilus-derived amylomaltase aqueous solution was added to obtain an enzyme-added starch liquid (25 g). The Thermus thermophilus-derived amylomaltase aqueous solution was added immediately before performing gelatinization and enzyme reaction of starch using RVA.

[Preparation of glucose-added starch liquid of Comparison plot 10-4]

As shown in Table 16, potato starch (manufactured by Nacalai Tesque, Inc.) was measured in an aluminum cup for RVA, glucose was added, and water (Milli-Q water) was added to obtain a glucose addition starch liquid (25 g).

[Preparation of enzyme and glucose-added starch liquids of Experimental plot 10-1 and Experimental plot 10-2]

As shown in Table 16, potato starch (manufactured by Nacalai Tesque, Inc.) was measured in an aluminum cup for RVA, glucose was added, water (Milli-Q water) was added, and diluted Thermus thermophilus-derived amylomaltase aqueous solution was added to obtain an enzyme and glucose-added starch liquid (25 g). The Thermus thermophilus-derived amylomaltase aqueous solution was added immediately before performing gelatinization and enzyme reaction of starch using RVA.

The amounts of the materials (Thermus thermophilus-derived amylomaltase aqueous solution, potato starch, glucose, water) used for the preparation of each liquid of the above-mentioned Experimental plot 10-1 and Experimental plot 10-2, Comparison plots 10-1 to 10-4 are shown in the following Table 16. In the Table, "TtAM" is amylomaltase derived from Thermus thermophilus.

[Table 16]

| | Comparison plot | | | | Experimental plot | |
|---|---|---|---|---|---|---|
| | 10-1 | 10-2 | 10-3 | 10-4 | 10-1 | 10-2 |
| diluted TtAM aqueous solution | | 0.1 mL (unit number per 1 g of potato starch: 1U) | 1 mL (unit number per 1 g of potato starch: 10U) | | 0.1 mL (unit number per 1 g of potato starch: 1U) | 1 mL (unit number per 1 g of potato starch: 10U) |
| potato starch | 1.25 g | | | | | |
| glucose | | 62.5 | | 62.5 mg | 62.5 mg | 62.5 mg |
| water | rest | | | | | |
| total amount | 25 g | | | | | |

Using RVA (manufactured by Perten Instruments, RVA-TecMaster), gelatinization and enzyme reaction of starch were performed for each liquid of Experimental plot 10-1 and Experimental plot 10-2, and Comparison plots 10-1 to 10-4 under the conditions shown in the following Table 17.

[Table 17]

| <RVA conditions> | | |
|---|---|---|
| time | temperature | rotating speed |
| 0 - 10 sec | maintained at 37°C | 960 rpm |
| 10 sec - 1 min | maintained at 37°C | 160 rpm |
| 1 min - 5 min | 37°C→98°C | |
| 5 min - 15 min | maintained at 98°C | |
| 15 min - 19 min | 98°C→50°C | |
| 19 min - 20 min | maintained at 50°C | |

The obtained respective reaction liquids were dispensed into 96-well plates by 300 μL (n=3) and the absorbance at a wavelength of 530 nm was measured using a microplate reader (manufactured by Molecular Devices Japan).

Each liquid after absorbance measurement was stored in a refrigerator (4°C) for 3 days, during which the absorbance at wavelength 530 nm was measured in the same manner as described above.

The results are shown in the following Table 18.

[Table 18]

| <absorbance (turbidity) at wavelength 530 nm> | | | | | | | |
|---|---|---|---|---|---|---|---|
| | | Comparison plot | | | | Experimental plot | |
| | | 10-1 | 10-2 | 10-3 | 10-4 | 10-1 | 10-2 |
| storage days | 0 | 0.07 | 0.07 | 0.07 | 0.08 | 0.07 | 0.05 |
| | 1 | 1.22 | 1.23 | 1. 67 | 1.17 | 0.48 | 0.06 |
| | 2 | 1.41 | 1.58 | 1.76 | 1.37 | 0.87 | 0.07 |
| | 3 | 1.51 | 1.73 | 1.8 | 1.47 | 1.12 | 0.08 |

As is clear from the results shown in Table 18, turbidity (absorbance at wavelength 530 nm) increased as storage days became longer in Comparison plot 10-1, and it was confirmed that starch was retrograded with time.

In Comparison plot 10-2 and Comparison plot 10-3 in which an amylomaltase derived from Thermus thermophilus was added alone, and Comparison plot 10-4 in which glucose was added alone, the degree of increase in the turbidity (absorbance at wavelength 530 nm) did not change, or turbidity increased more as compared with Comparison plot 10-1, and a retrogradation suppressive effect could not be confirmed.

On the other hand, in Experimental plot 10-1 and Experimental plot 10-2 in which an amylomaltase derived from Thermus thermophilus and glucose were used in combination, an increase in the turbidity was suppressed as compared with the control, and a retrogradation suppressive effect was obtained.

<Experimental Example 13>

(Comparison plot 11-1)

To commercially available dried potato flakes (manufactured by Kabushiki Kaisha Taimou, trade name "JAGAIMO Flake") (40 g) was added hot water (80°C) to a total amount of 160 g, and the mixture was stirred with a spatula for 3 min to give a uniform mixture. The mixture was divided into 3 packs of 50 g each, vacuum-packed, and heated in a water bath at 75°C for 30 min. After removing the rough heat with running water, the packs were refrigerated at 4°C for 1 day.

(Comparison plot 11-2)

By the same procedure as in Comparison plot 11-1 except that 100-fold diluted Thermus thermophilus-derived amylomaltase aqueous solution (146.5 μL) (unit number of amylomaltase derived from Thermus thermophilus per 1 g of dried potato flakes: 0.1U) was also added when hot water was added to commercially available dried potato flakes to make the total amount 160 g, mashed potato was prepared.

(Comparison plot 11-3)

By the same procedure as in Comparison plot 11-1 except that α-glucosidase (manufactured by Amano Enzyme Inc., "α-glucosidase "Amano"") (20 mg) (unit number of α-glucosidase per 1 g of dried potato flakes: 0.06U) was also added when hot water was added to commercially available dried potato flakes to make the total amount 160 g, mashed potato was prepared.

(Experimental plot 11-1)

By the same procedure as in Comparison plot 11-1 except that 100-fold diluted Thermus thermophilus-derived amylomaltase aqueous solution (146.5 μL) (unit number of amylomaltase derived from Thermus thermophilus per 1 g of dried potato flakes: 0.1U) and α-glucosidase (manufactured by Amano Enzyme Inc., "α-glucosidase "Amano"") (20 mg) (unit number of α-glucosidase per 1 g of dried potato flakes: 0.06U) were also added when hot water was added to commercially available dried potato flakes to make the total amount 160 g, mashed potato was prepared.

The amounts of amylomaltase derived from Thermus thermophilus and α-glucosidase (unit number per 1 g of dried potato flakes) used for the preparation of each mashed potato of Experimental plot 11-1 and Comparison plots 11-1 to 11-3 are shown in the following Table 19. In the Table, "TtAM" is Thermus thermophilus-derived amylomaltase, and "AG" is α-glucosidase.

[Table 19]

|  | Comparison plot | | | Experimental plot 11-1 |
| --- | --- | --- | --- | --- |
|  | 11-1 | 11-2 | 11-3 | |
| TtAM (unit per 1 g of dried potato flakes) |  | 0.1 |  | 0.1 |
| AG (unit per 1 g of dried potato flakes) |  |  | 0.06 | 0.06 |

The hardness of each of the mashed potatoes of Experimental plot 11-1 and Comparison plots 11-1 to 11-3 was measured using a texture analyzer (manufactured by Stable Micro Systems, model number: TA-XT Plus) and by the following procedures (1) - (2).

(1) the prepared mashed potato is dispensed to the edge of a 24-well flat-bottomed microplate (12 mm in diameter, 15 mm in height), covered with a lid, and stored at 4°C for 7 days.
(2) Using a stainless steel spherical plunger with a diameter of 5 mm, the center of the mashed potato filled in a 24-well flat-bottomed microplate is compressed (pierced) by 50% at a compression rate of 0.5 mm/sec, and the stress 1 second after compression is recorded.

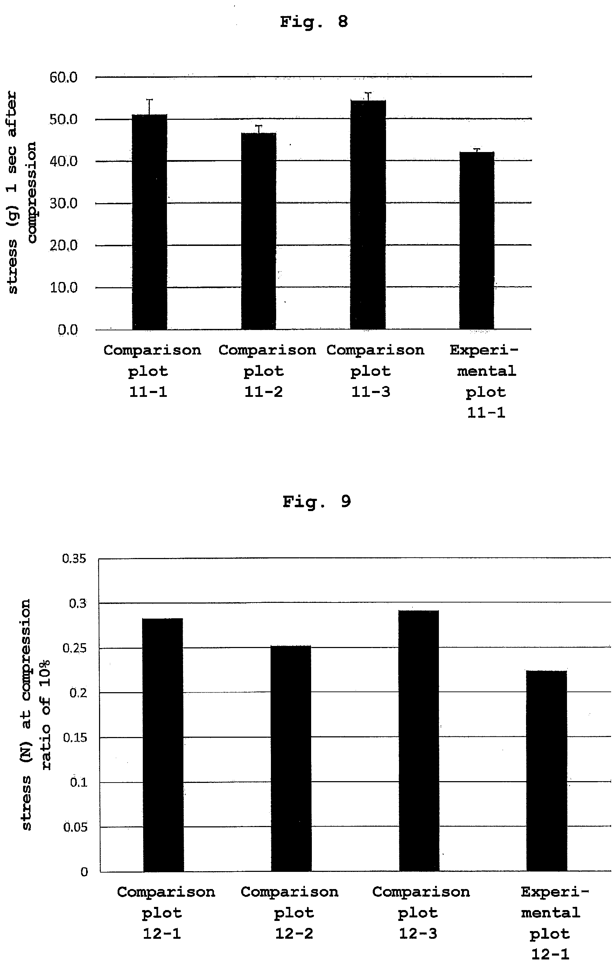

The results (hardness of mashed potato of Experimental plot 11-1 and Comparison plots 11-1 to 11-3 after refrigerated storage for 8 days) are shown in Fig. 8.
As is clear from the results shown in Fig. 8, the mashed potato of Experimental plot 11-1 using Thermus thermophilus-derived amylomaltase and α-glucosidase in combination was the softest after refrigerated storage for 8 days as compared with the Comparison plot 11-2 added with Thermus thermophilus-derived amylomaltase alone and the Comparison plot 11-3 added with α-glucosidase alone. The results suggest that a synergistic retrogradation suppressive effect is obtained by a combined use of Thermus thermophilus-derived amylomaltase and α-glucosidase.

<Experimental Example 14>

[Production of white bread]

(Comparison plot 12-1)

Bread flour (manufactured by Nippon Flour Mills Co., Ltd., trade name "NIPPN EAGLE (bread flour)"), granulated sugar (manufactured by Mitsui Sugar Co., Ltd., trade name "SPOON brand Granulated sugar"), and cooking salt (manufactured by Naikai Salt Industries Co., LTD., trade name "Nakuru M") were premixed in the amounts shown in the following Table 20 to give a mixed powder.

After weighing 190 g of city water (22.3°C) in a container (bread case with blades) of a commercially available automatic home bakery (automatic bread-baking machine for home use) (manufactured by MK Seiko Co., Ltd., product number: HBK-100), the above-mentioned mixed powder was added, 3 g of dry yeast (manufactured by Nisshin Foods Inc., trade name "Nissin Super Kameriya Dry Yeast") and 20 g of shortening (manufactured by J-OIL MILLS, Inc., trade name "FASHIE") were added on the mixed powder so as not to touch the city water. The menu of the automatic home bakery was set to "white bread/baked color: normal" (cooking time: 3 hr 50 min), and the start key was pressed to start cooking. The buzzer sounded to notify the end of cooking, the cancel key was pressed, the baked white bread was removed from the container, and the bread was allowed to cool at room temperature (24.5°C, humidity: 56%) for 1 hr.

[Table 20]

| material | weight (g) |
| --- | --- |
| bread flour | 280 |
| granulated sugar | 15 |
| cooking salt | 4 |

(Comparison plot 12-2)

By the same procedure as in Comparison plot 12-1 except that 1U of Thermus thermophilus-derived amylomaltase aqueous solution was added per 1 g of bread flour before a mixed flour was put into a container of a commercially available automatic home bakery, white bread was produced.

(Comparison plot 12-3)

By the same procedure as in Comparison plot 12-1 except that 0.75U of $\alpha$-amylase (manufactured by Amano Enzyme Inc., trade name "Biozyme A") per 1 g of bread flour was also added when a mixed powder was put into the container of a commercially available automatic home bakery, white bread was produced.

(Experimental plot 12-1)

By the same procedure as in Comparison plot 12-2 except that 0.75U of $\alpha$-amylase (manufactured by Amano Enzyme Inc., trade name "Biozyme A") per 1 g of bread flour was also added when a mixed powder was put into the container of a commercially available automatic home bakery, white bread was produced.

(Sensory evaluation)

After allowing to cool at room temperature (20°C) for 1 hr, the white breads of Experimental plot 12-1 and Comparison plots 12-1 to 12-3 were each sliced to a thickness of 2 cm, tightly sealed in a polyethylene bag, and stored at temperature of 10°C, humidity 50% for 2 days. After storage for 2 days, an evaluation panel of 4 people ate only the crumb part (internal phase part of white bread) of each white bread, and retrogradation suppressive effect, crispness and meltability in a mouth were each evaluated based on the following criteria. The sensory evaluation was performed by a disgorging method.

[Evaluation criteria of retrogradation suppressive effect, crispness and meltability in mouth]

⊙: drastically more preferable than Comparison plot 12-1
○: more preferable than Comparison plot 12-1
△: same level as Comparison plot 12-1

The results are shown in the following Table 21. In the Table, "TtAM" is an amylomaltase derived from Thermus thermophilus.

[Table 21]

| | Comparison plot | | | Experimental plot 12-1 |
|---|---|---|---|---|
| | 12-1 | 12-2 | 12-3 | |
| TtAM (unit per 1 g of bread flour) | | 1 | | 1 |
| $\alpha$-amylase (unit per 1 g of bread flour) | | | 0.75 | 0.75 |
| retrogradation suppressive effect | standard | ○ | △ | ⊙ |
| crispness | standard | △ | △ | ○ |
| meltability in mouth | standard | △ | ○ | ○ |
| comment | | slightly moist | soft but surface was dry | moist feeling was clearly improved, fluffy feeling was strong |

(Property measurement)

The white breads of Experimental plot 12-1 and Comparison plots 12-1 to 12-3 were tightly sealed in a polyethylene bag, stored for 2 days under the conditions of temperature 10°C, humidity 50%, and subjected to a compression test similar to that in Experimental Example 3. The results are shown in Fig. 9.

As is clear from the results shown in Table 21, all of retrogradation suppressive effect, crispness and meltability in a mouth were fine in the white bread of Experimental plot 12-1.

From the results shown in Fig. 9, it was confirmed that softness was maintained in the white bread of Experimental plot 12-1 as compared with the white bread of Comparison plot 12-2 in which an amylomaltase derived from Thermus thermophilus was added alone, and the white bread of Comparison plot 12-3 in which $\alpha$-amylase was added alone.

From these results, it was confirmed that a combined use of an amylomaltase derived from Thermus thermophilus and $\alpha$-amylase affords bread imparted with a moist feeling and softness and having preferable texture.

<Experimental Example 15>

(Comparison plot 13-1)

To commercially available dried potato flakes (manufactured by Kabushiki Kaisha Taimou, trade name "JAGAIMO Flake") (40 g) was added hot water (80°C) to a total amount of 160 g, and the mixture was stirred with a spatula for 5 min to give a uniform mixture. The mixture was divided into 3 packs of 50 g each, vacuum-packed, and heated in a water bath at 75°C for 30 min. After removing the rough heat with running water, the packs were refrigerated at 4°C for 1 day.

(Comparison plot 13-2)

By the same procedure as in Comparison plot 13-1 except that 100-fold diluted Thermus thermophilus-derived amylomaltase aqueous solution (73.3 μL) (unit number of amylomaltase derived from Thermus thermophilus per 1 g of dried potato flakes: 0.05U) was also added when hot water was added to commercially available dried potato flakes to make the total amount 160 g, mashed potato was prepared.

(Comparison plot 13-3)

By the same procedure as in Comparison plot 13-1 except that $\alpha$-amylase (manufactured by Amano Enzyme Inc., trade name "Biozyme A") (0.1 mg) (unit number of $\alpha$-amylase per 1 g of dried potato flakes: 0.0375U) was also added when hot water was added to commercially available dried potato flakes to make the total amount 160 g, mashed potato was prepared.

(Experimental plot 13-1)

By the same procedure as in Comparison plot 13-1 except that 100-fold diluted Thermus thermophilus-derived amylomaltase aqueous solution (73.3 μL) (unit number of amylomaltase derived from Thermus thermophilus per 1 g of dried potato flakes: 0.05U) and α-amylase (manufactured by Amano Enzyme Inc., trade name "Biozyme A") (0.1 mg) (unit number of α-amylase per 1 g of dried potato flakes: 0.0375U) were also added when hot water was added to commercially available dried potato flakes to make the total amount 160 g, mashed potato was prepared.

The amounts of amylomaltase derived from Thermus thermophilus and α-glucosidase (unit number per 1 g of dried potato flakes) used for the preparation of each mashed potato of Experimental plot 13-1 and Comparison plots 13-1 to 13-3 are shown in the following Table 22. In the Table, "TtAM" is Thermus thermophilus-derived amylomaltase.

[Table 22]

| | Comparison plot | | | Experimental plot 13-1 |
|---|---|---|---|---|
| | 13-1 | 13-2 | 13-3 | |
| TtAM (unit per 1 g of dried potato flakes) | | 0.05 | | 0.05 |
| α-amylase (unit per 1 g of dried potato flakes) | | | 0.0375 | 0.0375 |

The hardness of each of the mashed potatoes of Experimental plot 13-1 and Comparison plots 13-1 to 13-3 was measured using a texture analyzer (manufactured by Stable Micro Systems, model number: TA-XT Plus) and by the following procedures (1) - (2).

(1) the prepared mashed potato is dispensed to the edge of a 24-well flat-bottomed microplate (12 mm in diameter, 15 mm in height), covered with a lid, and stored at 5°C.
(2) Using a stainless steel spherical plunger with a diameter of 5 mm, the center of the mashed potato filled in a 24-well flat-bottomed microplate is compressed (pierced) by 50% at a compression rate of 1 mm/sec, and the stress 1 second after compression is recorded.

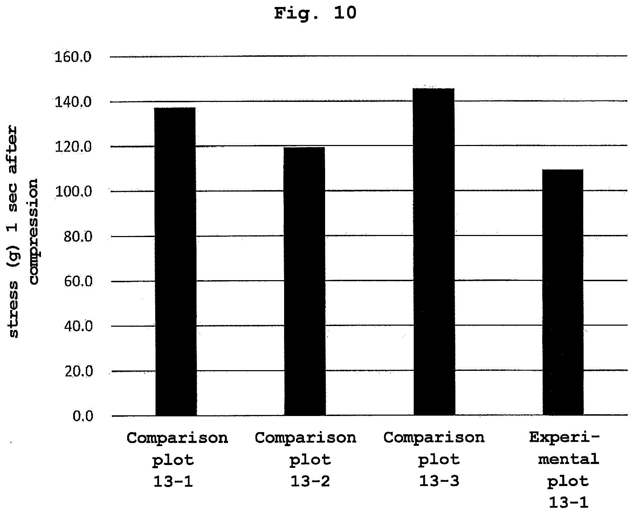

The results 8 days after the start of storage are shown in Fig. 10.

As is clear from the results shown in Fig. 10, the mashed potato of Experimental plot 13-1 using Thermus thermophilus-derived amylomaltase and α-amylase in combination was the softest after refrigerated storage for 8 days as compared with the Comparison plot 13-2 added with Thermus thermophilus-derived amylomaltase alone and the Comparison plot 13-3 added with α-amylase alone. The results suggest that a synergistic retrogradation suppressive effect is obtained by a combined use of Thermus thermophilus-derived amylomaltase and α-amylase.

[Industrial Applicability]

According to the present invention, methods for producing a modified starch-containing food (e.g., a method for producing a starch-containing food with suppressed retrogradation, a method for producing a starch-containing food with suppressed retrogradation and improved texture, a method for producing a starch-containing food with improved production suitability and improved texture, a method for producing a starch-containing food with improved flavor, etc.) can be provided.

In one embodiment according to the present invention, retrogradation of a starch-containing food can be suppressed, and therefore, according to the present invention, a method for producing a starch-containing food with suppressed retrogradation and a method for suppressing retrogradation of a starch-containing food can be provided.

In another embodiment according to the present invention, retrogradation of a starch-containing food can be suppressed, and texture can be improved. According to the present invention, therefore, a method for producing a starch-containing food with suppressed retrogradation and improved texture, a method for suppressing retrogradation of a starch-containing food, and a method for improving texture of a starch-containing food can be provided.

In another embodiment according to the present invention, production suitability of a starch-containing food can be improved and the texture can be improved. According to the present invention, therefore, a method for producing a starch-containing food with improved production suitability and improved texture, and a method for improving production suitability of a starch-containing food can be provided.

In another embodiment according to the present invention, a method for producing a starch-containing food with improved flavor, and a method for improving a flavor of a starch-containing food can be provided.

According to the present invention, an enzyme composition that can be preferably used for modifying a starch-containing food can be provided.

In one embodiment according to the present invention, an enzyme composition that can be preferably used for suppressing retrogradation of a starch-containing food and/or improving texture of a starch-containing food can be provided.

In another embodiment according to the present invention, an enzyme composition that can be preferably used for improving production suitability of a starch-containing food and/or improving texture of a starch-containing food can be provided.

In another embodiment according to the present invention, an enzyme composition that can be preferably used for improving a flavor of a starch-containing food can be provided.

This application is based on patent application Nos. 2019-002862 filed in Japan (filing date: January 10, 2019) and 2019-069262 filed in Japan (filing date: March 29, 2019), the contents of which are incorporated in full herein.

**Claims**

1. A method for producing a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

2. The production method according to claim 1, wherein an amount of the aforementioned (A) to be added to the starch-containing material is 0.0001 - 1000U per 1 g of the starch-containing material.

3. The production method according to claim 1 or 2, wherein the bacterium of the genus Thermus is at least one selected from the group consisting of Thermus thermophilus, Thermus lacteus, Thermus rubens and Thermus ruber.

4. The production method according to any one of claims 1 to 3, wherein the protein or lipid modification enzyme is at least one selected from the group consisting of glucose oxidase, transglutaminase, protease, protein asparaginase, lipase and phospholipase.

5. The production method according to any one of claims 1 to 4, wherein the starch degradation product is at least one selected from the group consisting of glucose, maltose, maltotriose and dextrin, and
the starch degradation enzyme is at least one selected from the group consisting of $\alpha$-amylase, $\beta$-amylase, glucoamylase and $\alpha$-glucosidase.

6. The production method according to any one of claims 1 to 5, further comprising adding (E) a protein modification enzyme to the starch-containing material in addition to the aforementioned (A), and (C) or (D).

7. The production method according to claim 6, wherein the protein modification enzyme is at least one selected from the group consisting of glucose oxidase and transglutaminase.

8. The production method according to any one of claims 1 to 7, wherein the starch-containing food is selected from the group consisting of bakery food, rice food, potato food, dough sheet food and rice-cakes.

9. An enzyme composition comprising (A) an amylomaltase derived from a bacterium of the genus Thermus, and (B) a protein or lipid modification enzyme, or (C) a starch degradation product or (D) a starch degradation enzyme.

10. The enzyme composition according to claim 9 for use for a starch-containing food.

11. The enzyme composition according to claim 10 for addition to the starch-containing material such that an amount of the aforementioned (A) to be added to the starch-containing material is 0.0001 - 1000U per 1 g of the starch-containing material.

12. The enzyme composition according to any one of claims 9 to 11, wherein the bacterium of the genus Thermus is at least one selected from the group consisting of Thermus thermophilus, Thermus lacteus, Thermus rubens and Thermus ruber.

13. The enzyme composition according to any one of claims 9 to 12, wherein the protein or lipid modification enzyme is at least one selected from the group consisting of glucose oxidase, transglutaminase, protease, protein asparaginase, lipase and phospholipase.

**14.** The enzyme composition according to any one of claims 9 to 13, wherein the starch degradation product is at least one selected from the group consisting of glucose, maltose, maltotriose and dextrin, and
the starch degradation enzyme is at least one selected from the group consisting of $\alpha$-amylase, $\beta$-amylase, glucoamylase and $\alpha$-glucosidase.

**15.** The enzyme composition according to any one of claims 9 to 14, further comprising (E) a protein modification enzyme in addition to the aforementioned (A), and (C) or (D).

**16.** The enzyme composition according to claim 15, wherein the protein modification enzyme is at least one selected from the group consisting of glucose oxidase and transglutaminase.

**17.** The enzyme composition according to any one of claims 10 to 16, wherein the starch-containing food is selected from the group consisting of bakery food, rice food, potato food, dough sheet food and rice-cakes.

**18.** The enzyme composition according to any one of claims 10 to 17 for modifying a starch-containing food.

**19.** The enzyme composition according to any one of claims 10 to 18 for suppressing retrogradation of the starch-containing food.

**20.** The enzyme composition according to any one of claims 10 to 19 for improving texture of the starch-containing food.

**21.** A method for modifying a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

**22.** A method for suppressing retrogradation of a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

**23.** A method for improving texture of a starch-containing food, comprising adding (A) an amylomaltase derived from a bacterium of the genus Thermus to the starch-containing material, and
further adding (B) a protein or lipid modification enzyme, or (C) a starch degradation product or (D) a starch degradation enzyme to the starch-containing material.

## Fig. 1

flask culture

↓

bacteria collection, washing

↓

cell disruption

(pressure homogenizer)

↓

supernatant of cell disruption liquid

↓

supernatant of heat-treated liquid

↓

ammonium sulphate fraction

↓

phenyl sepharose fraction

## Fig. 2

## Fig. 3

## Fig. 4

Fig. 5

## Fig. 6

values are means ±SD (n=5-9)

## Fig. 7

values are means ±SD (n=4-6)

Fig. 8

Fig. 9

Fig. 10

## INTERNATIONAL SEARCH REPORT

| | International application No. |
|---|---|
| | PCT/JP2020/000558 |

### A. CLASSIFICATION OF SUBJECT MATTER

Int. Cl. A23L5/00(2016.01)i, A23L7/10(2016.01)i, A23L7/109(2016.01)i, A23L19/10(2016.01)i, A23L19/12(2016.01)i, A23L29/00(2016.01)i, A21D2/26(2006.01)i, A21D8/04(2006.01)i, C12N9/10(2006.01)i
FI: A23L5/00 J, A23L5/00 N, A21D8/04, A23L7/10 E, A23L19/10, A23L7/109 A, C12N9/10, A23L29/00, A21D2/26, A23L19/12 Z, A23L7/109 B, A23L5/00 K

According to International Patent Classification (IPC) or to both national classification and IPC

### B. FIELDS SEARCHED

Minimum documentation searched (classification system followed by classification symbols)

Int. Cl. A23L5/00-5/30, 29/00-29/10, A23L19/00-19/20, A23L7/00-7/104, A21D2/00-17/00, A23L7/109-7/113, C12N9/00-9/99

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/FSTA/WPIDS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2012/111326 A1 (GLICO FOODS CO., LTD.) 23 August 2012, claims, paragraphs [0020]-[0022], [0033]-[0047], [0055], [0107]-[0141], [0153]-[0234] | 1-23 |
| Y | JP 11-332452 A (NOF CORP.) 07 December 1999, claims, paragraphs [0001], [0006]-[0015] | 1-23 |
| Y | JP 2002-272357 A (KANEKA CORP.) 24 September 2002, claims, paragraphs [0001], [0008], [0018]-[0022], [0029]-[0035] | 1-23 |
| Y | JP 7-31396 A (TORIGOE SEIFUN KK) 03 February 1995, claims | 1-23 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.03.2020 | 17.03.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2020/000558 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 6-292505 A (KANEKA CORP.) 21 October 1994, claims, paragraphs [0001], [0011], [0014]-[0018], [0034]-[0051] | 1-23 |
| Y | WO 2010/035858 A1 (AJINOMOTO CO., INC.) 01 April 2010, claims, paragraphs [0001]-[0003], [0006]-[0015] | 1-23 |
| Y | WO 2010/090337 A1 (AJINOMOTO CO., INC.) 12 August 2010, claims, paragraphs [0001]-[0003], [0005]-[0011] | 1-23 |
| Y | JP 57-166945 A (NIKKEN CHEMICALS CO., LTD.) 14 October 1982, claims, page 5, pages 7, 8 | 1-23 |
| Y | JP 7-79689 A (SAN-EI GEN F.F.I., INC.) 28 March 1995, claim 3, paragraphs [0001], [0003], [0020] | 1-23 |
| Y | JP 3-251173 A (AMANO PHARMA CO., LTD.) 08 November 1991, page 2, lower right column, lines 2-10 | 1-23 |
| Y | JP 9-322725 A (NIPPON SUISAN KAISHA LTD.) 16 December 1997, paragraphs [0002], [0003] | 1-23 |
| Y | JP 2011-115157 A (KOJO SEIFUN KK) 16 June 2011, paragraph [0030] | 1-23 |
| A | JP 2003-512046 A (NOVOZYMES A/S) 02 April 2003, entire text | 1-23 |
| A | JP 11-46780 A (EZAKI GLICO CO.) 23 February 1999, entire text | 1-23 |

Form PCT/ISA/210 (continuation of second sheet) (January 2015)

INTERNATIONAL SEARCH REPORT
Information on patent family members

International application No.

PCT/JP2020/000558

| Patent Documents referred to in the Report | Publication Date | Patent Family | Publication Date |
|---|---|---|---|
| WO 2012/111326 A1 | 23.08.2012 | (Family: none) | |
| JP 11-332452 A | 07.12.1999 | (Family: none) | |
| JP 2002-272357 A | 24.09.2002 | (Family: none) | |
| JP 7-31396 A | 03.02.1995 | (Family: none) | |
| JP 6-292505 A | 21.10.1994 | (Family: none) | |
| WO 2010/035858 A1 | 01.04.2010 | US 2011/0200706 A1 claims, paragraphs [0001]-[0028], [0031]-[0067] | |
| WO 2010/090337 A1 | 12.08.2010 | US 2012/0009298 A1 claims, paragraphs [0002]-[0046], [0048]-[0078] | |
| JP 57-166945 A | 14.10.1982 | (Family: none) | |
| JP 7-79689 A | 28.03.1995 | (Family: none) | |
| JP 3-251173 A | 08.11.1991 | (Family: none) | |
| JP 9-322725 A | 16.12.1997 | (Family: none) | |
| JP 2011-115157 A | 16.06.2011 | (Family: none) | |
| JP 2003-512046 A | 02.04.2003 | WO 01/029195 A1 entire text | |
| JP 11-46780 A | 23.02.1999 | EP 0884384 A2 entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H1146780 A
- WO 2012111326 A
- JP 2002272357 A
- JP H11332452 A
- JP 2001333712 A
- JP 2019002862 A
- JP 2019069262 A

### Non-patent literature cited in the description

- *Journal of Applied Glycoscinece,* 2011, vol. 1 (4), 281-285
- *Applied and Environmental Microbiology,* 2012, vol. 78 (20), 7223-7228
- **THIJS KAPER et al.** *Biochemistry,* 2007, vol. 46, 5261-5269